Europäisches Patentamt

European Patent Office

Office européen des brevets

(11) Veröffentlichungsnummer: **0 238 441 B1**

(12) **EUROPÄISCHE PATENTSCHRIFT**

(45) Veröffentlichungstag der Patentschrift: **10.11.93**

(21) Anmeldenummer: **87810135.1**

(22) Anmeldetag: **09.03.87**

(51) Int. Cl.⁵: **C12N 15/32**, C12P 21/02, A01N 63/00, C12N 1/18, C12N 1/20

(54) **Insektizid wirksame proteinartige Substanz.**

(30) Priorität: **15.03.86 GB 8606441**

(43) Veröffentlichungstag der Anmeldung:
**23.09.87 Patentblatt 87/39**

(45) Bekanntmachung des Hinweises auf die
Patenterteilung:
**10.11.93 Patentblatt 93/45**

(84) Benannte Vertragsstaaten:
**AT BE CH DE ES FR GR IT LI LU NL SE**

(56) Entgegenhaltungen:
**EP-A- 0 063 949        EP-A- 0 093 062**
**EP-A- 0 100 561        EP-A- 0 200 344**
**EP-A- 0 228 838        WO-A-86/01536**

**PROC. NATL. ACAD. SCI. USA, Band 78, Nr. 5,
Mai 1981, Seiten 2893-2897; E. SCHNEPF:
"Cloning and expression of the Bacillus thuringiensis crystal protein gene in Escherichia coli"**

(73) Patentinhaber: **CIBA-GEIGY AG**
**Klybeckstrasse 141**
**CH-4002 Basel(CH)**

(72) Erfinder: **Geiser, Martin, Dr.**
**Hauptstrasse 3A**
**CH-4107 Ettingen(CH)**
Erfinder: **Hinnen, Albert, Dr.**
**Offenburgerstrasse 20**
**CH-4057 Basel(CH)**
Erfinder: **Brassel, Jakob, Dr.**
**1157/56 Okamoto Bairin**
**Higashinada-ku 658 Kobe(JP)**
Erfinder: **Schweitzer-Grützmacher, Silvia, Dr.**
**Hasenhain 16**
**D-6915 Dossenheim(DE)**

CHEMICAL ABSTRACTS, Band 103, 1985, Seite 155, Zusammenfassung Nr. 17768z, Columbus, Ohio, US; H.E. SCHNEPF et al.: "Delineation of a toxin-encoding segment of a Bacillus thuringiensis crystal protein gene"

GENE, Band 36, Nr. 3, 1985, Seiten 289-300, Elsevier, Amsterdam, NL; M.J. ADANG et al.: "Characterized full-length and truncated plasmid clones of the crystal protein of Bacillus thuringiensis subsp. Kurstaki HD-73 and their toxicity to Manduca sexta"

GENE, Band 48, 1986, Seiten 109-118, Elsevier Science Publishers B.V., Amsterdam, NL; M. GEISER et al.: "The hypervariable region in the genes coding for entomopathogenic crystal proteins of Bacillus thuringiensis: Nucleotide sequence of the Kurhd 1 gene of subsp. Kurstaki HD1"

## Beschreibung

Die vorliegende Erfindung betrifft DNA-Fragmente enthaltend eine für ein insektizidwirksames Toxinprotein kodierende DNA-Sequenz oder ausgewählte Teile davon; die nach Expression besagter DNA Sequenz bzw. besagter Teilsequenzen erhältlichen Toxinproteine; insektizide Mittel enthaltend als aktiven Bestandteil mindestens eines der besagten Toxinproteine; sowie deren Verwendung in einem Verfahren zur Bekämpfung von Insekten.

Ebenso umfasst von der vorliegenden Erfindung sind Mikroorganismen, insbesondere Bakterien und Hefen, welche das erfindungsgemässe DNA-Fragment oder Teile davon einkloniert enthalten.

Bacillus thuringiensis (B. thuringiensis) ist ein gram-positives Bakterium, das in der Regel für Insekten pathogen ist (S. Chang[1]).

Die verschiedenen Stämme von B. thuringiensis unterscheiden sich dabei beträchtlich hinsichtlich ihrer Toxizität und ihres Wirkspektrums für Insekten. Die insektizide Aktivität von B. thurigiensis stammt im wesentlichen oder auch vollständig von einem proteinartigen parasporalen Kristallkörper, der zum Zeitpunkt der Sporulation in der Wachstumsphase gebildet wird. Das (die) Gen(e), das (die) für die toxischen Proteine (Polypeptide) des erwähnten Kristallkörpers kodiert (kodieren), wurde(n) auf Plasmid-DNA und/oder auf chromosomaler DNA des B. thuringiensis gefunden.

Um jegliche Nachteile zu vermeiden, die sich aufgrund der Anwesenheit anderer von B. thuringiensis produzierter Komponenten ergeben könnten, und um das insektizid wirksame Polypeptid in grosser Menge zu erhalten, ist es vorteilhaft, das entsprechende Gen, d.h. die entsprechende DNA-Sequenz, welche(s) für das gewünschte insektizid wirksame Protein kodiert, unabhängig von B. thuringiensis zu verwenden.

Dennoch ist es auch möglich und unter gewissen Umständen vorteilhaft, B. thuringiensis selbst mit der erwähnten DNA-Sequenz zu transformieren.

Auf diese Weise ist es möglich ein Protein zu erhalten, das in seiner Struktur und seinen Eigenschaften dem natürlichen Produkt analog ist.

Aus Schnepf et al.[4], Adang et al.[3], Shibano et al.[30] und Schnepf et al.[32] sowie aus WO 86/01536 sind bereits Toxin-kodierende Gene aus B. thuringiensis bekannt. Ebenfalls bekannt sind chimäre Toxin-Moleküle, welche aus Teilsequenzen des HD-73-Gens aus B. thuringiensis HD-73 sowie des HD-1 Gens aus B. thuringiensis HD-1 zusammengesetzt sind [EP-A 0 228 838]

Die Aufgabe, die es im Rahmen der vorliegenden Erfindung zu lösen galt, betraf somit in erster Linie das Auffinden neuer, insektizid-wirksamer Toxinproteine und der diese Toxinproteine kodierenden DNA-Sequenzen sowie die Identifizierung der entsprechenden Aminosäure- und Nukleotidsequenzen.

Diese Aufgabe konnte im Rahmen der vorliegenden Erfindung mit Hilfe zum Teil bekannter Verfahrensmassnahmen gelöst werden, durch Bereitstellung eines Verfahrens zur Herstellung einer insektizid wirksamen proteinartigen Substanz einschliesslich der Entdeckung und Identifizierung der korrespondierenden, vollständigen DNA Sequenz.

Die vorliegende Erfindung betrifft somit ein Verfahren zur Herstellung einer insektizid wirksamen proteinartigen Substanz einschliesslich der Entdeckung und Identifizierung der vollständigen DNA-Sequenz, die für das insektizid wirksame Protein MGE 1 besagter proteinartiger Substanz kodiert und sich von den bereits bekannten B. thuringiensis Genen (H.E. Schnepf et al.[4);32], M.J. Adang et al.[3] und Shibano et al.[30] sowie WO 86/01536 und EP-A 0 228 838) deutlich unterscheidet.

Die vorliegende Erfindung betrifft weiterhin ein DNA-Fragment, das durch die in Tabelle 2 wiedergegebene Nucleotid-Sequenz charakterisiert ist und eine für ein insektizid wirksames Toxinprotein kodierende DNA Sequenz umfasst, einschliesslich ausgewählter Teile besagten DNA Fragments, mit der Massgabe, dass besagte ausgewählte Teile einen kodierenden Anteil enthalten, der ausreicht, damit die insektizide Aktivität des korrespondierenden Proteinfragments erhalten bleibt und dass besagte ausgewählte Teile isoliert und nicht als Bestandteil einer chimären Genkonstruktion aus zwei oder mehreren *Bacillus thuringiensis* Genen vorliegen.

Das Protein (Polypeptid), das man im Rahmen der vorliegenden Erfindung erhält, wird im Folgenden als MGE 1 bezeichnet.

Bevorzugt im Rahmen der vorliegenden Erfindung ist ein ausgewählter Teil besagten DNA Fragments, welcher eine Grösse von 4355 Bp aufweist und innerhalb des Klons pK36 vorliegt, der einkloniert in *E.coli* HB101 unter der Hinterlegungsnummer DSM 3668 hinterlegt worden ist und durch Schneiden mit HpaI und PstI aus diesem erhältlich ist.

Ebenfalls bevorzugt ist ein ausgewählter Teil besagten DNA Fragments, der dadurch gekennzeichnet ist, dass besagtes Teilfragment für das insektizid wirksame Protein MGE 1 kodiert, einschliesslich ausgewählter Teile davon, mit der Massgabe, dass die insektizide Aktivität bei den durch besagte ausgewählte Teile kodierten Toxinfragmenten erhalten bleibt und dass besagte ausgewählte Teile isoliert und nicht als

3

Bestandteil einer chimären Genkonstruktion aus zwei oder mehreren *Bacillus thuringiesis* Genen vorliegen.

Die vorliegende Erfindung betrifft ferner eine proteinartige Substanz, die zumindest teilweise durch eines der zuvor genannten, anmeldungsgemässen DNA Fragmente kodiert wird oder durch ausgewählte Teile davon, mit der Massgabe, dass nur dieser besagte Teil der proteinartigen Substanz aus *Bacillus thuringiensis* stammt.

Besonders bevorzugt im Rahmen dieser Erfindung ist das Protein MGE 1, welches durch die in Tabelle 3 wiedergegebene Aminosäuresequenz charakterisiert ist.

Unter dem Begriff "proteinartige Substanz" soll sowohl das insektizid wirksame Protein MGE 1 selbst als auch in vitro erhältliche Derivate und Modifikationen davon verstanden werden, wie beispielsweise das Protein MGE 1 in Verbindung mit anderen Protein-Fragmenten, in erster Linie solchen, die von anderer klonierter und für andere pestizide, vorzugsweise insektizide Aktivitäten kodierender DNA stammen, wobei besagte Proteinkombinationen als "Fusionsproteine" eingestuft werden.

Pestizide Aktivität beinhaltet neben insektizider Aktivität beispielsweise auch bakterizide, virizide, fungizide und herbizide Aktivität, vorzugsweise gegenüber pflanzenpathogenen Organismen. Von den proteinartigen Substanzen ist das insektizid wirksame Protein MGE 1 für sich allein bevorzugt.

Die vorliegende Erfindung betrifft somit auch Fusionsproteine, wobei es sich bei dem aus *Bacillus thuringiensis* stammenden Teil besagter Fusionsproteine um das Protein MGE 1 handelt, während der Teil der Fusionsproteine, der vom Protein MGE 1 verschieden ist, insbesondere eine herbizide oder insektizide Aktivität besitzt.

Ein weiterer Gegenstand der vorliegenden Erfindung betrifft ein Verfahren zur Konstruktion von Klonierungs- und Expressions-Vehikeln, die das in Tabelle 2 wiedergegebene DNA-Fragment enthalten sowie besagte Vehikel selbst. Geeignete DNA-Vektoren sind beispielsweise Plasmide wie pBR322 und pUC8 oder Phagen wie M13.

Weiterhin bezieht sich die vorliegende Erfindung auf lebende oder tote Mikroorganismen, die eines der zuvor erwähnten, anmeldungsgemässen DNA-Fragmente enthalten, das durch die in Tabelle 2 wiedergegebene Nukleotid-Sequenz charakterisiert ist, vorzugsweise auf einen Mikroorganismus der Spezies Saccharomyces cerevisiae.

Die Erfindung bezieht sich weiterhin auf Mikroorganismen, insbesondere auf Mikroorganismen der Spezies Saccharomyces cerevisiae, die einen ausgewählten Teil besagten DNA Fragments enthalten, welcher eine Grösse von 4355 Bp aufweist und innerhalb des Klons pK36 vorliegt, der einkloniert in *E.coli* HB101 unter der Hinterlegungsnummer DSM 3668 hinterlegt worden ist und durch Schneiden mit HpaI und PstI aus diesem erhältlich ist und für eine insektizid wirksame proteinartige Substanz, einschiesslich ausgewählter Teile davon, kodiert, unter der Voraussetzung, dass die insektizide Aktivität besagter ausgewählter Teile erhalten bleibt.

Bei besagten Mikroorganismen handelt es sich beispielsweise um Hefen, vorzugsweise Saccharomyces cerevisiae, Bakterien und auf Blattoberflächen angesiedelte Pilze, mit der Einschränkung, dass besagter Mikroorganismus, falls er zur Gruppe des Bacillus thuringiensis gehört, zuvor mit einem der zuvor erwähnten, anmeldungsgemässen DNA-Fragmente transformiert worden ist, wie es in Tabelle 2 wiedergegeben ist. Unter "Transformation" sollen in diesem Zusammenhang auch konjugationsähnliche Mechanismen verstanden werden.

Die Erfindung beinhaltet ausserdem ein Bioenkapsulierungssystem, das aus einem ersten Material besteht, welches vollständig in einem zweiten Material biologischen Ursprungs eingebettet vorliegt, wobei es sich bei dem ersten Material um eines der zuvor erwähnten, anmeldungsgemässen DNA-Fragmente entsprechend der Beschreibung in Tabelle 2 handelt, bei dem zweiten Material um einen vollständigen Mikroorganismus mit der Einschränkung, dass besagter Mikroorganismus, falls er zur Gruppe des Bacillus thuringiensis gehört, zuvor mit besagtem DNA-Fragment transformiert worden ist.

Bei dem DNA-Material kann es sich insbesondere um einen ausgewählten Teil besagten DNA Fragments handeln, welcher eine Grösse von 4355 Bp aufweist und innerhalb des Klons pK36 vorliegt, der einkloniert in *E.coli* HB101 unter der Hinterlegungsnummer DSM 3668 hinterlegt worden ist und durch Schneiden mit HpaI und PstI aus diesem erhältlich ist, und für eine insektizid wirksame proteinartige Verbindung einschliesslich ausgewählter Teile davon kodiert, unter der Voraussetzung, dass die insektizide Aktivität besagter ausgewählter Teile erhalten bleibt. Besonders geeignete Mikroorganismen sind Hefen, vorzugsweise Saccharomyces cerevisiae, wie Saccharomyces cerevisiae GRF 18.

Ein weiterer Bestandteil der vorliegenden Erfindung betrifft ein Mittel sowie ein Verfahren zur Bekämpfung von Insekten, vorzugsweise lepidopteren Insekten (Insekten der Ordnung Lepidoptera), in erster Linie Vertreter der Gattungen Pieris, Heliothis, Spodoptera und Plutella, wie z.B. Pieris brassicae, Heliothis virescens, Heliothis zea, Spodoptera littoralis, Plutella xylostella sowie verwandte Arten, mit Hilfe der proteinartigen Substanz, enthaltend das Protein MGE 1, das durch die in Tabelle 2 wiedergegebene DNA Sequenz codiert wird.

Das Verfahren ist gekennzeichnet durch die Applikation einer insektizid wirksamen Menge einer proteinartigen Substanz, die zumindest teilweise durch das anmeldungsgemässe DNA-Fragment kodiert wird, welches für das Protein MGE 1 kodiert, direkt auf die Insekten oder ihr Verbreitungsgebiet. Das Mittel beinhaltet eine insektizid wirksame Menge einer proteinartigen Substanz, die zumindest teilweise durch das anmeldungsgemässe DNA-Fragment kodiert wird, welches für das Protein MGE 1 kodiert.

Darüberhinaus betrifft die vorliegende Erfindung eine Applikationsform, die aus transformierten lebenden oder toten Hefezellen besteht, die eine insektizid wirksame Menge einer proteinartigen Substanz enthalten, die zumindest teilweise durch das anmeldungsgemässe DNA-Fragment kodiert wird, welches für das Protein MGE 1 kodiert, wobei besagte Applikationsform für das Ausbringen der aktiven Substanz in einer geschützten Form geeignet ist.

Es ist daher eine lang anhaltende insektizide Aktivität erreichbar, wenn die transformierten Hefezellen auf konventionelle Art und Weise appliziert werden, wie z.B. durch Aufsprühen auf das Feld (Boden- und Luftapplikation). Die aktive Verbindung ist gut geschützt gegen vorzeitigen Abbau aufgrund ungünstiger Bedingungen, wie beispielsweise Sonneneinstrahlung oder widrige Verhältnisse auf den Blattoberflächen.

Das klonierte Gen kann unter die Kontrolle eines Hefe-Promotors gestellt und exprimiert werden, wobei die insektizide Aktivität sowohl a) für den Extrakt als auch in b) für ganze Zellen nachweisbar ist.

Geeignete Hefe-Promotoren sind in der Europäischen Patentanmeldung 100 561 beschrieben. Besonders geeignet ist der PHO5 Promotor.

Zumindest von einigen der Bacillus thuringiensis Gene, die für insektizid wirksame Proteine kodieren, ist bekannt, dass sie mit einem Promotor verbunden sind, der von der Escherichia coli (E.coli) RNA Polymerase erkannt werden kann, wobei besagter Promotor vor dem jeweiligen Gen lokalisiert ist (H.C. Wong et al.[2])).

Das Verfahren zur Herstellung einer insektizid wirksamen proteinartigen Substanz im Rahmen der vorliegenden Erfindung beinhaltet die Transkription und Translation eines Gens mit identifizierter DNA-Sequenz entsprechend der vorliegenden Erfindung, in das entsprechende Protein mit Hilfe von E.coli oder Hefe.

Das hier beschriebene Verfahren zur Gewinnung von Ausgangsmaterial wird unter Verwendung von Bacillus thuringiensis var. kurstaki HD1, Stamm ETHZ 4449, durchgeführt. Dieser Stamm kann von der mikrobiologischen Abteilung der Eidgenössischen Technischen Hochschule in Zürich, Schweiz, bezogen werden und ist für jedermann ohne irgendwelche Beschränkung frei zugänglich.

Der Ursprung dieses Stammes ist die H. Dulmage Collection an der Cotton Insects Research Unit, US. Department of Agriculture, Agriculture Research Center, in Brownsville, Texas, wo er ebenfalls für jedermann frei zugänglich ist.

Gemäss vorliegender Erfindung kann die für das Protein MGE 1 kodierende DNA erhalten werden durch

a) Isolierung und Lyse von *Bacillus thuringiensis* var. *kurstaki* HD1-Zellen, Abtrennen der Plasmide von dem so erhaltenen lysierten Material mit Hilfe bekannter Massnahmen und Reinigen und Dialysieren des so erhaltenen Plasmid-Materials;

b) Erstellung einer DNA-Bibliothek der *Bacillus thuringiensis* var. *kurstaki* HD1 Plasmid-DNA;

c) Klonieren der gemäss Schritt b) erhaltenen fragmentierten Plasmid DNA in einem geeigneten Vektor;

d) Screening der Klone auf die Anwesenheit von Antigen, das mit Antikörpern reagiert, die gegen das Kristallkörper-Protein von *B. thuringiensis* var. *kurstaki* hergestellt worden sind;

e) Auslesen der Klone, die spezifisch mit besagten Antikörpern reagieren; und

f) Prüfung der insektiziden Aktivität von Extrakten der gemäss Schritt e) erhaltenen Klone.

Die Identifizierung der DNA kann nach an sich bekannten Methoden durchgeführt werden, wie sie beispielsweise unter den Punkten h) und i) aufgeführt sind:

h) Kartierung der DNA positiver Klone durch Verdauung mit Restriktionsendonucleasen und Hybridisierung der so erhaltenen Fragmente mit radioaktiv markierter RNA; und

i) Sequenzierung von DNA-Fragmenten, die für die jeweiligen Proteine codieren.

Bedeutung der im Folgenden verwendeten Abkürzungen:

| | |
|---|---|
| Bp: | Basenpaare |
| BSA: | Rinder-Serum-Albumin (bovine serum albumin) |
| DEAE: | Diethylaminoethyl |
| DFP: | Diisopropylfluorphosphat |
| DTT: | 1,4-Dithiothreitol(1,4-dimercapto-2,3-butandiol) |
| EDTA: | Ethylendiamintetraessigsäure |
| IPTG: | Isopropyl-$\beta$-thiogalactopyranosid (Serva) |

Kb: Kilobasen
PSB[1]: 0,01 M Phosphat Puffer, pH 7.4 und 0,8 % NaCl
PSB[2]: 10 mM Natriumphosphat, pH 7.8 und 0,14 % NaCl
PEG 6000: Polyethylenglykol mit mittlerem Molekulargewicht 6000
PMSF: Phenylmethylsulfonylfluorid (Fluka)
RT: Raumtemperatur
SDS: Natriumdodecylsulfat
STE: siehe TNE
TBS: 10 mM Tris•HCl, pH 7.5 und 0,14 M NaCl
TE: Lösung enthaltend 10 mM Tris•HCl (pH 7.5) und 1 mM EDTA
TES: 0,5 M Tris, pH 8.0, 0,005 M NaCl und 0,005 M EDTA
TNE: Lösung enthaltend 100 mM NaCl, 10 mM Tris•HCl (pH 7.5) und 1 mM EDTA
Tris•HCl: Tris-(hydroxymethyl)-aminomethan, pH-Einstellung mit HCl
X-GAL: 5-Brom-4-chlor-3-indoxyl-$\beta$-D-galaktosid
2xYT: 16 g Bacto Trypton,
10 g Hefe Extrakt (Bacto),
5 g NaCl

Im Folgenden verwendete Medien, Puffer und Lösungen:

| 20 x SSC | Lösen von 175,3 g NaCl und 88,2 g Natriumcitrat in 800 ml $H_2O$. Einstellen des pH auf einen Wert von pH 7.0 mit einigen Tropfen einer 10 N NaOH-Lösung. Einstellen des Volumens auf 1 Liter; Aufteilen der Lösung in Aliquots; Sterilisation durch Autoklavieren. |
|---|---|
| 2 x SSC | 10 % von 20xSSC |
| 6 x SSC | 33 % von 20xSSC |

| Denhardt's-Lösung (50 x) | Ficoll 70 [relative Molekülmasse ca. 700'000; Pharmacia] | 5 g |
|---|---|---|
| | Polyvinylpyrrolidon | |
| | (Calbiochem-Behring Corp.) | 5 g |
| | BSA (Sigma) | 5 g |
| | $H_2O$ | auf 500 ml |
| Filtration durch einen Nalgene®-Einmalfilter (Nalgene®; Nalge Co. Inc., Rochester, N.Y., USA). Auftrennen in 25 ml Aliquots und Aufbewahren bei -20°C. | | |

```
Lösungen

M9 Medium:      Pro Liter:

          a) Na₂HPO₄               6 g

             KH₂PO₄               3 g

             NaCl              0,5 g

             NH₄Cl                1 g

          Einstellung des pH-Wertes auf 7.4, autoklavieren,

          abkühlen und anschliessend hinzufügen von:
```

b) 1 M MgSO₄      2 ml

20 % Glukose      10 ml

1 M CaCl₂      0,1 ml

Vitamin B1 (40 mg/10 ml)      5 ml

Die oben genannten Lösungen a) und b) sollten getrennt sterilisiert werden durch Filtration (Glukose, Vit. B1) oder durch Autoklavieren.

| LB (Luria-Bertani) Medium | Pro Liter: | |
|---|---|---|
| | Bacto-Trypton | 10 g |
| | Bacto-Hefe-Extrakt | 5 g |
| | NaCl | 10 g |

Einstellen des pH-Wertes auf 7.5 mit Natriumhydroxid.

L-Broth      siehe LB-Medium

Für die Induktion der Sporulation bei B. thuringiensis var. kurstaki, wird ein GYS-Medium entsprechend den Angaben von Yousten und Rogoff (A.A. Yousten und M.H. Rogoff[5]) (1969) verwendet (g/l⁻¹):

| Glukose | 1 |
|---|---|
| Hefe-Extrakt (Difco) | 2 |
| $(NH_4)_2SO_4$ | 2 |
| $K_2HPO_4$ | 0,5 |
| $MgSO_4 \cdot 7H_2O$ | 0,2 |
| $CaCl_2 \cdot 2H_2O$ | 0,08 |
| $MnSO_4 \cdot H_2O$ | 0,05 |

Vor dem Autoklavieren wird der pH-Wert mit Kaliumhydroxid auf einen Wert von 7.3 eingestellt.

Charakterisierung der im Rahmen der vorliegenden Erfindung verwendeten Mikroorganismen:

1. HD1-ETHZ 4449 ist ein Bacillus thuringiensis Stamm der Subspezies kurstaki, der zum einen durch seine immunologische Reaktion gegen sein Flagellum-Antigen charakterisiert ist - HD1-ETHZ 4449 gehört zum 3a, 3b Serotyp (A. Krieg[31]) - zum anderen durch sein spezifisches Southern blot-Muster, das man bei Durchführung der Southern-blot-Experimente, wie sie unter Punkt III 5.b beschrieben sind, erhält.

Die Gesamt-DNA dieses Stammes wird isoliert und mit dem Restriktionsenzym Hind III vollständig verdaut; die so erhaltenen Fragmente werden dann nach ihrer Grösse auf einem Agarose-Gel aufgetrennt und anschliessend auf ein Nitrocellulose-Papier überführt. Das radioaktiv markierte EcoRI-Fragment Pos. 423 bis Pos. 1149 (Tabelle 2) hybridisiert spezifisch mit 3 Fragmenten, die eine Grösse von 6,6 Kb, 5,3 Kb bzw. 4,5 Kb aufweisen.

2. Bei HB 101 handelt es sich um ein Hybrid zwischen Escherichia coli K12 x Escherichia coli B. Dieser Stamm ist gut geeignet für gross angelegte DNA-Reinigungen. Es wird verwendet für Transformationsexperimente und CaCl₂-kompetente Zellen sind kommerziell erhältlich z.B. bei Gibco AG, Basel, Schweiz, Katalog-Nr. 530 8260 SA.

3. Bei JM 103 handelt es sich um ein Escherichia coli K-12, das z.B. bei Pharmacia P-L Biochemicals kommerziell erhältlich ist, Katalog-Nr. 27-1545-xx (1984).

Die E.coli-Stämme HB 101 und JM 103 sind bei Maniatis et al. (T. Maniatis et al.[6]) beschrieben:

| Stamm | Genotyp |
|---|---|
| HB 101 | $F^-$, hsdS$_{20}$ ($r_B^-$, $m_B^-$), recA$_{13}$, ara-14, proA$_2$, lacY$_1$, galK$_2$, rpsL$_{20}$ (Sm$^r$), xyl-5, mtl-1, supE$_{44}$, $\lambda^-$ |
| JM 103 | $\Delta$ (lac pro), thi, strA, supE, endA, sbcB, hsdR, F'traD36, proAB, lacI$^q$, Z$\Delta$M15 |

Im folgenden Beispiel ist der Ausdruck "Hefe" gleichbedeutend mit Saccharomyces cerevisiae.

Beispiele

I. Plasmid-DNA Aufarbeitung:

Die Aufarbeitung der Plasmid-DNA erfolgt wie unten beschrieben entsprechend den Angaben bei White und Nester (F.F. White und E.W. Nester[7]).

I.1. B. thuringiensis Plasmide

B. thuringiensis var. kurstaki HD1 4479 Zellen werden in 1 Liter LB-Medium unter Schütteln 12-14 Stunden bei 30°C kultiviert und entsprechend den Angaben bei White und Nester (F.F. White und E.W. Nester[7]) aufgearbeitet. Nach der Ernte werden die Zellen in einem alkalischen lysierenden Puffer resuspendiert und bei einer Temperatur von 37°C für 20-30 Minuten inkubiert. Man erhält ein klares Lysat, das durch Zugabe von 2 M Tris•HCl (pH 8) neutralisiert wird. Die chromosomale DNA wird anschliessend durch Zugabe von SDS und NaCl präzipitiert. Das Lysat wird auf Eis gepackt und die chromosomale DNA durch Zentrifugation entfernt. Die Plasmid-DNA, die sich jetzt im Ueberstand befindet, wird mit 10 % PEG 6000 präzipitiert. Nach der Aufbewahrung der Plasmid DNA über Nacht bei 4°C erfolgt die Resuspendierung in 7-8 ml TE. Die aus 1 Liter Kultur-Lösung gewonnene Plasmid DNA wird anschliessend über 2 CsCl-Gradienten weiter gereinigt. Es wird dabei festes CsCl zu der Lösung hinzugegeben (8,3 g CsCl auf 8,7 ml Ueberstand). Nach dem Hinzufügen von Ethidiumbromid (Sigma; Endkonzentration 1 mg/ml Ueberstand) wird die Lösung in 13,5 ml 'Quick Seal' Polyallomer-Röhrchen (Beckmann) überführt und in einem Beckmann Ti50-Rotor für einen Zeitraum von 40 Stunden bei 40'000 rpm zentrifugiert. Unter langwelligem UV-Licht (366 nm) werden zwei fluoreszierende Banden sichtbar gemacht. Die untere Bande enthält überspiralige Plasmid-DNA, die durch seitliches Punktieren des Zentrifugenröhrchens mit einer 2 ml Spritze (18 G Nadel) gesammelt wird. Ethidiumbromid wird durch 5-maliges Waschen mit gleichen Volumina Isopropanol (gesättigt mit CsCl) entfernt und das Produkt dann in 30 ml Corex-Röhrchen überführt. Es werden 2,5 Volumen TE hinzugefügt und anschliessend wird die DNA mit Ethanol präzipitiert. Diese Lösung wird dann für 12-15 Stunden bei -20°C aufbewahrt. Die präzipitierte DNA wird anschliessend durch Zentrifugation in einem Sorvall HB-4 Rotor über einen Zeitraum von 30 min bei 12'000 rpm und einer Temperatur von 0°C gesammelt und in 200 $\mu$l TE gelöst. (E.coli JM 103 kann ebenso extrahiert und in analoger Weise behandelt werden).

I.2. E.coli Plasmide

Die Zellen einer 100 ml Kultur (LB-Medium) werden durch Zentrifugation (Sorvall, GSA Rotor, 10 min bei 6'000 rpm, 4°C) gesammelt, in 100 ml TE (10 mM Tris•HCl, 1 mM EDTA, ph 8.0) resuspendiert und unter den gleichen Bedingungen erneut zentrifugiert. Das Zell-Pellet wird anschliessend in 3 ml Tsuc [50 mM Tris•HCl, pH 7.5, 25 % (w/v) Sucrose] resuspendiert und in SS-34 Polypropylen Sorvall Röhrchen überführt. Alle nachfolgenden Schritte werden auf Eis durchgeführt: Zunächst werden 0,3 ml einer Lysozym-Lösung (10 mg/ml, bezogen von Worthington, 11'000 U/mg) nach 5 min 1,2 ml EDTA (500 mM, pH 8.0) und nach weiteren 5 min 4,8 ml Detergenz zugegeben [0,1 % Triton X-100 (Merck), 50 mM EDTA, 50 mM Tris•HCl, pH 8,0]. Nach 5 Minuten wird das Lysat in einem vorgekühlten SS-34 Rotor für 40 Minuten bei 4°C zentrifugiert. Der Ueberstand wird vorsichtig entfernt und nach Zugabe von festem CsCl, entsprechend der für die B. thuringiensis Plasmid DNA gemachten Angaben, über einem CsCl-Gradienten gereinigt.

Aus 100 ml Kulturlösung werden 50-100 $\mu$g Hybrid-Plasmid DNA gewonnen.

## II. $\delta$-Endotoxin Antigen und Ziegen-Antikörper

### II.1. Herstellung von $\delta$-Endotoxin Kristallkörper-Antigen:

Bacillus thuringiensis (var. kurstaki HD 1, Stamm ETHZ 4449) wird in einer Fernbach-Flasche auf einem Medium nach Yousten und Rogoff, (A.A. Yousten und M.H. Rogoff[5]) wie zuvor beschrieben, jedoch mit einem erhöhten Glukoseanteil (0,3 % anstatt 0,1 %), kultiviert.

Die Inkubationszeit beträgt 4-5 Tage bei einer Temperatur von 30°C. Die Kolonien werden unmittelbar nach erfolgter Sporulation (B. Trümpi[8]) geerntet. Zur Trennung von Sporen und Parasporalkörpern wird die Methode von Delafield et al. (F.P. Delafield et al.[9]) verwendet.

### a) Trennung von Sporen und Kristallkörper:

- Suspendieren autolysierter Kulturen in 1M NaCl/0.02 M Kaliumphosphat-Puffer (pH 7.0) mit 0.01 % Triton-X-100 (Merck).
- Filtrieren der Suspension zur Abtrennung particulärer Bestandteile, wie Agar-Reste u.a..
- Zentrifugieren.
- Mehrmaliges Waschen des Sediments mit der oben beschriebenen Lösung, bis nur noch Spuren von Bestandteilen, die bei 260 nm absorbieren, im Ueberstand vorhanden sind.
- Waschen der particulären Bestandteile in 0.2 M NaCl 0.004 M Phosphat-Puffer (pH 7.0)/0.01 % Triton-X-100.
- Wiederholen des Waschvorgangs mit 0.01 % Triton-X-100.
- Resuspendieren der Partikel in Wasser.
- Entfernen der restlichen Zellen aus der Suspension.
- 3-maliges Zentrifugieren und anschliessendes Waschen der zurückgebliebenen Sporen und Kristalle in 0.02 M Phosphat-Puffer (pH 7.0)/0.01 % Triton-X-100.
- Ueberführen der Suspension, in 182 ml desselben Puffers, der in einen zylindrischen Scheidetrichter, der 105 g einer 20 %igen (w/w) wässrigen Natriumdextransulfat 500-Lösung (Sigma), 13.2 g festes Polyethylenglykol 6000 (Merck), 3.3 ml 3 M Phosphat-Puffer (pH 7.0) sowie 7.5 g NaCl enthält.
- Schütteln zur Lösung der festen Bestandteile.
- Einstellen des Volumens auf 600 ml durch Zugabe einer gut durchmischten Lösung gleicher Zusammensetzung, aber ohne bakterielle Bestandteile.
- Kräftig schütteln.
- 30 Minuten bei 5°C stehen lassen.
- Nach erfolgter Phasentrennung Abziehen der oberen Phase (enthält den Grossteil der Sporen aber nur sehr wenig Kristalle).
- Zentrifugieren der oberen Phase.
- Ueberführen des Ueberstands in den Scheidetrichter zu der dort verbliebenen unteren Phase (enthält eine Mischung aus Sporen und Kristallen).
- Wiederholen des Extraktionsvorgangs.

Nach der 10ten Extraktion sind die Kristallkörper in der unteren Phase praktisch frei von Sporen und können durch Zentrifugation isoliert werden. Sowohl die Sporen, wie auch die Kristallkörper werden anschliessend 5 mal in kaltem destilliertem Wasser gewaschen. Die Kristallkörper werden als wässrige Suspension bei einer Temperatur von -5°C aufbewahrt.

### b) Lösen der Kristallkörper

Ein Aliquot der kristallkörperhaltigen Suspension wird für 10 Minuten bei 12'000 g zentrifugiert. Das so gewonnene Sediment wird in 0,05 M Carbonat-Puffer und 10 mM Dithiothreitol (DTT, Sigma; die Mischungen von Carbonat-Puffer und Dithiothreitol wird im folgenden als Carbonat/DTT bezeichnet) in einer Konzentration von 5 mg Sediment/ml Carbonat/DTT-Mischung resuspendiert.

Nach 30 minütiger Inkubation bei 37°C werden die unlöslichen Partikel durch 10 minütige Zentrifugation bei 25'000 g abgetrennt. Der Ueberstand wird gegen Carbonat-Puffer dialysiert und anschliessend auf seinen Proteingehalt und seine Aktivität im Biotest hin untersucht.

Für Aufbewahrungszwecke wird die Protoxinlösung in einzelne Portionen aufgeteilt, die tiefgefroren werden. Protein, das frei von DTT ist, hat beim Auftauen eine Gel-artige Konsistenz. Ein vollständiges Lösen des Proteins wird durch Zugabe von 1 mM DTT erreicht.

c) Inaktivierung Kristallkörper-gebundener Proteasen:

Serin Proteasen und Metall-Proteasen der Kristallkörpersuspension [Chestukhina et al. [10)]] werden durch Zugabe von Diisopropylfluorphosphat (DFP, Serva) und EDTA auf die im folgenden angegebenen Weise inaktiviert:

Die Kristallkörper werden in 0,01 M Phosphat-Puffer, pH 8,0 und 1 mM EDTA in einer Konzentration von 5-10 mg Kristallkörper/ml Puffer/EDTA-Mischung, suspendiert. Die Suspension wird dann mit Ultraschall behandelt, bis eine monodisperse Lösung vorliegt (dies wird mit Hilfe eines Lichtmikroskops überprüft).

In einem Abzug wird, unter den üblichen Sicherheitsvorkehrungen, 1 mM DFP Zu der Suspension zugegeben. Das Röhrchen, das die Suspension enthält, wird luftdicht verschlossen und kräftig geschüttelt. Nach Inkubation über Nacht bei Raumtemperatur wird die inaktivierte Suspension so lange dialysiert, bis ein Gleichgewicht gegenüber $H_2O$ und 1 mM EDTA erreicht ist.

## II.2. Immunisierung von Ziegen

Das Antigen wird hergestellt aus Kristallkörpern des B. thuringiensis Serotyp H-3 durch Auflösen der Kristallkörper in Carbonat/DTT, Dialysieren der so erhaltenen Lösung gegen Carbonat-Puffer und 1 mM DTT und Reinigen des Antigens durch Steril-Filtration unter Verwendung eines 0,45 $\mu$m Millipore Filters.

Die Antigen-Lösung wird mit "komplettem Freund's Adjuvants" (Bacto) in einem Verhältnis von 1:1 gemischt und bei 4°C gelagert.

In der Versuchsstation der CIBA-GEIGY AG in St. Aubin (Fribourg, Schweiz), werden 2 Ziegen mit H-3 Protoxin immunisiert. Jede der beiden Ziegen erhält ein intracutane Injektion von 0,5 mg Antigen und eine subcutane Injektion von 1,5 ml Pertussis (Behring), letzteres zur Steigerung der Immunreaktion. Die gesamte Behandlung wird an den Tagen 0, 28 und 76 durchgeführt. Blutproben werden am 35., 40., 84. und 89. Tag abgenommen, wobei am 35. Tag 5 ml und an den übrigen Tagen je 80 ml entnommen werden.

Nach Koagulation des Blutes werden die Seren zur Inaktivierung des Komplement-Systems für 30 Minuten bei 56°C inkubiert. Die Seren werden bei -20°C aufbewahrt.

## II.3. Reinigung und [$^{125}$I]-Markierung der Ziegen-H3 Antikörper

a) Reinigung des Immunglobulin:

Die IgG (Immunglobulin G) Fraktion des Ziegen $H_3$-Antiserums wird durch Ammoniumsulfat-Präzipitation gereinigt, gefolgt von einer Chromatographie an DEAE Cellulose und einer Analyse auf Ouchterlony Immundiffusionsplatten (O. Ouchterlony[11)]) entsprechend der von Huber-Lukac (H. Huber-Lukac[12)]) beschriebenen Methode.

Dabei werden 30 ml 3,2 M Ammoniumsulfat zu 15 ml Ziegen-$H_3$-Antiserumin 15 ml PBS[1] (0,01 M Phosphat-Puffer, pH 7.4 und 0,8 % NaCl) zugetropft. Die Mischung wird für 15 Minuten stehengelassen. Nach Zentrifugation der Mischung (10'000 g, 20 min), wird das Sediment in 7,5 ml PBS[1] wiederaufgenommen, dreimal bei einer Temperatur von 4°C gegen 1000 ml PBS[1] dialysiert, anschliessend gegen 1000 ml 0,01 M Phosphat-Puffer pH 7.8 dialysiert und zuletzt zentrifugiert (3000 g, 20 min).

Der Ueberstand wird auf eine 30 ml DEAE-Säule aufgetragen und bei RT mit 0,01 M Phosphatpuffer pH 7.8 eluiert (Durchflussrate: 20-80 ml/h). Die Fraktionen des ersten Peak werden gepoolt und lyophilisiert. Die durchschnittliche IgG-Ausbeute liegt bei 180 mg/15 ml Serum. Die Reinheit der IgG Fraktion wird mit Hilfe der Immundiffusion (O. Ouchterlony[11)]) gegen Anti-Ziegen-IgG Antikörper und gegen Anti-Ziegen-Serum Antikörper des Kaninchens (Miles Laboratories) überprüft.

Die weitere Reinigung der Antikörper erfolgt durch Absorption an einer Sepharose®(Pharmacia)Säule, die $H_3$-Protoxin gebunden enthält. Die Bindung des Protoxin an CNBr-Sepharose® (Pharmacia) wird entsprechend der vom Hersteller gemachten Angaben durchgeführt, die sich wie folgt zusammenfassen lassen:

1 g CNBr-aktivierte Sepharose® 6MB wird für ein Gel-End-Volumen von 3 ml abgewogen. Das Gel wird gewaschen und auf einer Glasfritte unter Verwendung von 200 ml 1 mM HCl erneut gequollen. Das $H_3$-Antigen Protein, das man wie oben unter Punkt II.1.c beschrieben erhält, wird in 0,1 M $NaHCO_3$ und 0,5 M NaCl gelöst. 1 ml des Gels enthält dann 5-10 mg Protein. Die Gel-Suspension und das Antigen werden für 2 Stunden bei Raumtemperatur vermischt. Das überschüssige Protein wird durch Waschen mit 0,1 M $NaHCO_3$ (pH 8.3), 0,5 M NaCl und 0,5 M Ethanolamin entfernt. Ein weiterer Waschvorgang schliesst sich an mit 0,1 M $NaHCO_3$ (pH 8.3) und 0,5 M NaCl, gefolgt von 0,1 M $CH_3COONa$ (pH 4) und 0,5 M NaCl. Der letzte Waschvorgang wird dann wieder mit 0,1 M $NaHCO_3$ und 0,5 M NaCl durchgeführt.

Das Protein-Sepharose®-Konjugat kann anschliessend in eine Pharmacia-K9-Säule (Pharmacia) gepackt werden. Das IgG kann dann an dieser Säule sehr effektiv gereinigt werden.

Spezifisch gebundene Antikörper werden mit 3 M KSCN eluiert und gegen $PBS^2$ (10 mM Natrium-Phosphat pH 7.8, 0,14 M NaCl) dialysiert. Die Antikörper werden anschliessend unter Verwendung der Chloramin-T Methode (Amersham Büchler Review[13]) mit $^{125}$I radioaktiv markiert.

b) Iod-Markierung:

1 m Ci Natrium-jodid-$^{125}$I wird in ein Röhrchen mit 100 $\mu$l 0,5 M Phosphat-Puffer (pH 7.2) gegeben. Unter ständigem Rühren werden 5 $\mu$g der Protein-Lösung (0,5 mg/ml in TBS), und 50 $\mu$g Chloramin T in 0,05 M Phosphat-Puffer (pH 7.2) hinzugefügt. Nach einminütiger Inkubation bei RT erfolgt die Zugabe von 120 $\mu$g $Na_2S_2O_5$. Freies Jod wird von dem markierten Protein über eine 0,9 x 12 cm Säule, gepackt mit Sephadex® G-25, abgetrennt. Um eine Absorption des markierten Proteins an die Säule zu verhindern, lässt man zunächst 0,5 ml BSA (100 mg/ml) durch die gepackte Säule laufen. Danach wird das markierte Material quantitativ auf die Säule überführt und mit Phosphatpuffer (pH 7.2) eluiert. Die Fraktionen (1 ml) werden gesammelt, bis das gesamte Protein eluiert ist.

III. Klonieren der $\delta$-Endotoxin-Gene

Eine partielle Sau3A-DNA-Bibliothek der B. thuringiensis var. kurstaki HD1, Stamm ETHZ 4449 Plasmid DNA im wesentlichen entsprechend den Angaben bei Maniatis et al. (T. Maniatis et al.[14]) hergestellt und in die BamHI-Restriktionsstelle von pBR322, das als Vektor DNA fungiert, subkloniert, wie unten beschrieben:

III.1. Partielle Verdauung hochmolekularer B. turingiensis DNA

Die Verdauung mit Sau3A wird in der Weise durchgeführt, dass die Anfärbung der DNA-Bruchstücke auf dem Agarose-Gel mit Ethidiumbromid bevorzugt im 2-10 Kb-Bereich erfolgt. Dies wird durch Anwendung der von Maniatis et al. (T. Maniatis et al.[14]) beschriebenen Methode erreicht.

Die Auftrennung der partiell zerstückelten DNA wird auf einem präparativen Agarose-Gel durchgeführt, wie es in Abschnitt IV.2. beschrieben ist oder vorzugsweise auf einem NaCl Salz-Gradienten. Man stellt den linearen Salz-Gradienten zwischen 5 und 20 % NaCl in TE-Puffer ein und zentrifugiert bei 35 Krpm für 3 Stunden in einem SW 40 Ti Beckman Rotor. Die gesammelten Fraktionen werden durch Zugabe von Ethanol präzipitiert und auf einem Agarose-Gel analysiert.

10 $\mu$g des Plasmids pBR322 werden mit 10 Einheiten der Endonuclease BamHI in 50 $\mu$l 10 mM Tris-HCl, pH 7.4, 100 mM NaCl und 10 mM $MgCl_2$ bei 37°C 1-2 h verdaut.

Die Phosphatase-Behandlung der gespaltenen DNA wird folgendermassen durchgeführt:

10 $\mu$g DNA werden zunächst in 50 $\mu$l Tris•HCl, pH 8, gelöst, anschliessend erfolgt die Zugabe von alkalischer Phosphatase aus Rinderdarm (Boehringer) in einer Konzentration von 3 Einheiten/$\mu$g DNA. Nach einer 30 minütigen Inkubation bei 37°C wird die DNA zweimal mit Phenol behandelt und anschliessend mit Chloroform extrahiert. Nach erfolgter Ethanol-Prezipitation wird die DNA in 20 $\mu$l $H_2O$ resuspendiert und für die Verknüpfungs-Reaktion mit den durch partielle Sau3A-Verdauung erhaltenen DNA-Bruchstücken verwendet. Die Reaktion wird folgendermassen durchgeführt: Zu 0,4 $\mu$g Sau3A verdauter DNA in 10 $\mu$l $H_2O$ werden 0,1 $\mu$g des Phosphatase behandelten Vektors hinzugegeben.

Die Verknüpfungsreaktion wird erreicht durch Zugabe von 50 mM Tris•HCl, pH 7.4, 1 mM ATP, 10 mM $MgCl_2$ und 15 mM DTT, mit nachfolgender Applikation von 20 Einheiten $T_4$ DNA Ligase (Biolabs). Nach einer Inkubation bei 15°C über Nacht wird die DNA zur Transformation von kompetenten E.coli HB101 Zellen verwendet.

Alternativ zur Herstellung einer partiellen Sau3A-Bibliothek kann auch eine DNA-Bibliothek von B. thuringiensis (var. kurstaki HD1, Stamm ETHZ 4449) durch vollständige BamHI und teilweise ClaI-Verdauung der Plasmid-DNA erstellt werden. Danach erfolgt die Klonierung in pBR322 zwischen den ClaI- und BamHI-Schnittstellen.

III.2. Transformation mit Hilfe des Calciumchlorid-Verfahrens:

Die Bereitstellung kompetenter Zellen erfolgt durch Behandlung von Zellen, die bis zu einer Populationsdichte von 5 x $10^7$ Zellen/ml herangewachsen sind, mit Calciumchlorid (Maniatis et al.[15]). Die Transformation wird erreicht durch Zugabe von DNA zu diesen Zellen. Anschliessend werden die Zellen 3 Minuten bei 42°C inkubiert, gefolgt von einer Verdünnung mit 1 ml LB-Medium, einer Inkubation während 60 Minuten bei 37°C sowie der Verteilung auf selektive Medien unter Verwendung allgemein bekannter Methoden (T. Maniatis et al[15]).

### III.3. Herstellen roher Zell-lysate

Die Kolonien, die das δ-Endotoxin-Gen enthalten, exprimieren ein Protein mit einer ähnlichen biologischen Aktivität wie die gereinigten und gelösten Toxin-Kristalle (H.E. Schnepf et al[16]). Sie werden daher mit Hilfe immunologischer Methoden unter Verwendung von Ziegen-Antikörpern (den H₃-Antikörpern), welche gegen B. thuringiensis var. kurstaki Kristallkörper-Protein hergestellt wurden, gescreent. Die Bakterienkolonien werden einzeln in 5 ml LB-Medium in Gegenwart von Ampicillin kultiviert. Zehn Kulturen werden jeweils gepoolt, geerntet und in 10 mM NaCl gewaschen; zuletzt werden die Zellen in 2 ml 400 mM NaCl, 0,1 M NaOH und 1 mM PMSF lysiert. Nach 20 minütiger Inkubation bei Zimmertemperatur werden die Lysate durch Zugabe von 20 µl 2 M Tris•HCl, pH 7.0, neutralisiert. Nach Zentrifugation in einem SS34 Sorvall Rotor (20 min, 10'000 rpm) werden die Lysate ausgiebig gegen TBS (10 mM Tris-HCl, pH 7.5, 0,14 M NaCl) dialysiert.

### III.4. Radioimmunologisches Screening der Zellextrakte

Die Extrakte werden mit Hilfe der Plastikbecher-Methode, wie sie bei Clarke et al. (L. Clarke et al.[17]) beschrieben ist, radioimmunologisch auf Anwesenheit von δ-Endotoxin-Antigen hin untersucht. Einzelne Plastikbecher werden über Nacht mit 150 µl gereinigten H3-Ziegen-Antikörpern (10 µg/ml) in 10 mM Tris•HCl, pH 9.3, beschichtet und über Nacht bei 4°C aufbewahrt. Die Becher werden dreimal mit TBS/Tween (TBS + 0.5 % Tween 20) gewaschen, mit 150 µl des Bakterienextrakts gefüllt und für 6 Stunden bei 37°C inkubiert. Nach dem Waschen werden die Becher mit 150 µl [$^{125}$I] markierten Kaninchen-Anti-Ziegen-H3-Antikörpern (60 ng, $10^5$ cpm] in TBS, enthaltend 25 % Pferdeserum, versetzen und über Nacht bei Zimmertemperatur inkubiert. Nach erneutem Waschen mit TBS/Tween werden die einzelnen Plastikbecher in einem Szintillationszähler gemessen.

### III.5. Restriktionskarte und Lokalisation des δ-Endotoxin Gens auf dem rekombinanten Plasmid pK19

a) Restriktionskartierung:

Die Restriktionskarte des DNA-Klons pK19, die man nach dem oben beschriebenen immunologischen Screening der Sau3A-Bibliothek des B. thuringiensis HD1, ETHZ 4449 erhält, ist aus den Ergebnissen einmaliger, zweimaliger und dreimaliger Verdauungen der Plasmid-DNA mit verschiedenen Restriktionsenzymen ableitbar. Die Enzymverdauungen werden alle entsprechend den Anweisungen des Herstellers durchgeführt. Kurzgesagt wird die DNA (1 µg /50 µl) zunächst in einem für die betreffenden Restriktionsendonucleasen geeigneten Puffer gelöst und anschliessend die verdaute DNA nach einer Inkubationszeit von 1-2 Stunden bei 37°C auf ein Agarose-Gel aufgetragen und einer Elektrophorese unterzogen. Falls eine weitere Behandlung mit einem zweiten Enzym nötig wird unter Bedingungen, welche inkompatibel mit dem ersten Enzym sind (beispielsweise aufgrund eines falschen Puffers), wird die DNA zunächst mit einer 1:1 Mischung von Phenol und Chloroform extrahiert, anschliessend mit Ethanol precipitiert und zuletzt unter den zuvor inkompatiblen Bedingungen (wie z.B. in einem Puffer, der für das 2. Enzym benötigt wird) inkubiert.

b) Southern transfer:

Die für das δ-Endotoxin kodierende Sequenz wird mit Hilfe der Hybridisierungsreaktion radioaktiv markierter RNA aus sporulierenden B. thuringiensis-Zellen, mit spezifischen Restriktionsfragmenten von pK19 bestimmt. Dies wird durch Anwendung der bei Southern (Southern [18]) beschriebenen Transfer-Technik erreicht:
Entsprechend ihrer Grösse über ein Agarose-Gel elektrophoretisch aufgetrennte DNA-Fragmente werden denaturiert, auf ein Nitrozellulosefilter übertragen und immobilisiert. Die relative Lage der DNA-Fragmente im Gel wird dabei im Verlaufe ihres Transfers auf den Filter beibehalten. Die an den Filter gebundene DNA wird anschliessend mit $^{32}$P-markierter RNA hybridisiert; durch Autoradiographie wird dann die Position jeder einzelnen Bande lokalisiert, die komplementär zu der radioaktiven Probe ist.
Der DNA-Transfer vom Agarose-Gel auf Nitrozellulose-Papier wird entsprechend den Anweisungen bei Maniatis et al.[19] durchgeführt.

c) Hybridisierung der Southern-Filter:

Die Vorhybridisierung und die eigentliche Hybridisierung werden entsprechend den Anweisungen bei Maniatis et al. (T. Maniatis et al.[20]) mit den folgenden Modifikationen durchgeführt: Die gebackenen Filter werden in einen durch Hitze verschweissbaren Plastikbeutel gegeben.

Pro $cm^2$ Nitrozellulosefilter werden 0,2 ml einer Prehybridisierungs-Mixtur zugegeben.

Prehybridisierungs-Mixtur:    4 x SSC

50 % Formamid

0,2 % SDS

20 mM EDTA

25 mM Kaliumphosphat (pH 7.2)

5 x Denhard's Lösung

100 $\mu$g/ml denaturierte Kalbsthymus-DNA

Die Beutel werden in der Regel für 3-4 Stunden bei 37°C inkubiert.

Die Prehybridisierungs-Mixtur wird entfernt und durch die folgende Hybridisierungs-Mixtur (50 $\mu$l/$cm^2$ Nitrozellulosefilter) ersetzt.

Hybridisierungs-Mixtur:    Gleiche Zusammensetzung wie die Prehybridisierungsmixtur, aber jetzt mit $^{32}$P-markierter denaturierter RNA Probe ($10^6$-$10^7$ cpm/Filter), hergestellt entsprechend den unten unter Punkt III.5.d. gemachten Angaben.

Die Beutel werden gewöhnlich bei 37°C über Nacht aufbewahrt. Nach der Hybridisierung werden die Filter 15 Minuten in 2 x SSC und 0,1 % SDS bei RT gewaschen, wobei besagter Waschvorgang zweimal wiederholt wird; anschliessend werden die Filter 60 Minuten in 0,1 x SSC und 0,1 % SDS gewaschen. Die Filter werden auf Whatman 3MM Papier getrocknet und für die Autoradiographie vorbereitet.

d) Isolation und radioaktive Markierung der B. thuringiensis var. kurstaki RNA.

B. thuringiensis var. kurstaki Zellen werden auf einem Rogoff-Medium, enthaltend 0,1 % Glucose (A.A. Yousten and M.H. Rogoff[5]) kultiviert. 500 ml Kulturen werden in 2 Liter-Erlenmeier-Flaschen bei 300 rpm und 30°C geschüttelt. Während des Zellwachstums geht der pH-Wert von pH 7 auf etwa 4.8 zurück und steigt dann wieder auf Werte von pH 7 an. Zu diesem Zeitpunkt beginnen die Zellen zu verklumpen. Der Zeitpunkt, an dem der pH seinen Ausgangswert wieder erreicht, wird als Startpunkt der Sporulation angesehen. Die Zellen werden für weitere 5 bis 6 Stunden kultiviert. Man fügt dann Rifampicin (50 $\mu$g/ml) hinzu und schüttelt die Zellen für weitere 10 Minuten. Die eisgekühlten Zellen werden geerntet und in 10 ml 4 M Guanidinthiocyanat, 0,5 % Sarcosyl, 25 mM Natriumcitrat pH 7 und 0,1 M 2-Mercaptoethanol resuspendiert. Die Zellen werden bei -80°C tiefgefroren und anschliessend in einer 'French Press' aufgebrochen. Nach einer 15 minütigen Zentrifugation des Zellextraktes bei 15'000 rpm (Sorvall SS34 Rotor) werden 0,5 g/ml CsCl zu dem Ueberstand hinzugegeben, der dann in einem Beckman 60Ti Zentrifugenröhrchen auf eine aus 5,7 M CsCl, 0,1 M EDTA bestehende Unterlage aufgeschichtet wird. Nach erneuter Zentrifugation für 20 h bei 38'000 rpm wird das RNA-Pellet mit Ethanol gewaschen, getrocknet, in 7 M Guanidinhydrochlorid gelöst und mit Ethanol prezipitiert. Die Gesamt-RNA aus sporulierenden Zellen wird dephosphoryliert und mit [$^{32}$P] ATP und T4 Polynukleotid-Kinase nach standardisierten Verfahren (N. Maizels[21]) markiert.

RNA Markierung mit Polynukleotid-Kinase:

Etwa 1 $\mu$g RNA wird durch Erhitzen in 50 mM Tris-HCl (pH 9.5) einer milden alkalischen Hydrolyse unterworfen; Zeit und Temperatur der Inkubation: 20 Minuten bei 90°C.

Die Hydrolyse liefert freie 5' Hydroxylgruppen, die als Substrate für die Polynukleotid-Kinase dienen. Die Hydrolyse wird in versiegelten Kapillarröhrchen mit einem Gesamtvolumen von 4 $\mu$l durchgeführt. Die Kinase Markierung erfolgt in Reaktionseinheiten von 10 $\mu$l, die 50 mM Tris-HCl (pH 9.5), 10 mM $MgCl_2$, 5 mM Dithiothreitol, 5 % Glycerol und 1 $\mu$M [$\gamma^{32}$P]-ATP, markiert mit einer spezifischen Aktivität von 6000 Ci/mmol, enthalten. Jede Reaktionseinheit enthält etwa 1 $\mu$g RNA und 2 $\mu$l T4 Polynukleotid Kinase, die Reaktion wird bei 37°C über einen Zeitraum von 45 Minuten durchgeführt. Es entsteht auf diese Weise eine RNA mit einer spezifischen Aktivität von ca. 3 x $10^7$ Cerenkov cpm/$\mu$g. Die RNA wird vom [$\gamma^{32}$P]-ATP durch dreimalige Ethanol Prezipitation in Gegenwart von 5 $\mu$g eines tRNA Carriers abgetrennt.

III.6. Identifizierung von Klonen, die für das δ-Endotoxin, in der BamHI/ClaI Plasmid DNA Bibliothek, kloniert in pBR322, codieren: Die pK 25 Serie

a) In situ Hybridisierung bakterieller Kolonien:

Die Koloniehybridisierung (M. Grunstein and D. Hogness[22]) wird durchgeführt durch Ueberführen der Bakterien von einer die Basiskultur enthaltenden Platte ("master plate") auf einen Nitrocellulosefilter. Die auf dem Filter befindlichen Kolonien werden anschliessend lysiert und die freigesetzte DNA wird durch Erhitzen auf dem Filter fixiert. Nach Hybridisierung mit einer $^{32}$P-markierten Probe wird der Filter mit Hilfe der Autoradiographie kontrolliert. Eine Kolonie, deren DNA bei der Autoradiographie ein positives Ergebnis bringt, kann dann von der die Basiskultur enthaltenden Platte gewonnen werden.

Für das Screening der BamHI/ClaI Plasmid DNA Bibliothek, kloniert in pBR322, wird ein innerhalb des δ-Endotoxins gelegenes DNA-Fragment verwendet. Die Methode ist bei Maniatis et al. (T. Maniatis et al.[23]) beschrieben.

Die Filter werden mit einer $^{32}$P-markierten Probe, die gemäss der nachfolgend unter Punkt III.6.b beschriebenen Methode hergestellt wird, hybridisiert.

Zur Herstellung eines Autoradiographiebildes wird der Filter in "Saran Wrap" eingeschlagen und einem Röntgenfilm ausgesetzt.

Die positiven Klone werden von der die Basiskultur enthaltenden Platte isoliert und analysiert. Sie besitzen neben einer DNA-Sequenz, die für das Toxin kodiert, die DNA flankierende Region, was anhand immunologischer Verfahren (siehe Punkt III.4. oben) sowie von Restriktionskartierungen (siehe Punkt III.5. oben) und in einem in vivo Biotest (siehe Punkt III.7. unten) nachgewiesen werden konnte. Diese Klone werden im folgenden mit pK25-i bezeichnet, wobei i für eine Zahl zwischen 1-7 steht.

b) DNA-Nick-Translation:

Eine Radionuklid-Markierung eines internen DNA-Fragments des δ-Endotoxin Gens wird nach dem im folgenden beschriebenen Verfahren durchgeführt:

Mixtur: 3 $\mu$l DNA ( 1$\mu$g)

1,5 $\mu$l Nick Translation Puffer (10-fach konzentriert: 0,5 M Tris, pH 8, 0,05 M MgCl$_2$)

1,5 $\mu$l 2,5 mM d Guanosin-5'-triphosphat (GTP)

1,5 $\mu$l 2,5 mM d Cytidin-5'-triphosphat (CTP)

1,5 $\mu$l 2,5 mM d Thymidin-5'-triphosphat (TTP)

2,5 $\mu$l H$_2$O

0,75 $\mu$l BSA (1 mg/ml)

1,5 $\mu$l 100 mM $\beta$-Mercaptoethanol

vermischen und zu 100 $\mu$Ci getrocknetem [$^{32}$P-$\alpha$]-ATP [10 mCi/mmol] hinzugeben

gut durchmischen,

0,75 $\mu$l einer 1 x 10$^{-4}$-Losung von DNAseI (1 mg/ml) in Nick Translations Puffer hinzufügen,

1 Minute bei RT inkubieren, auf Eis überführen

1 $\mu$l E.coli Polymerase I (Biolabs; Endvolumen: 15 $\mu$l) zugeben.

3 Stunden bei 15°C inkubieren,

bei 65°C 10 Minuten erhitzen, 35 $\mu$l 50 mM EDTA und 10 $\mu$l tRNA Stammlösung (100 $\mu$g) hinzufügen. Nicht eingebaute Nukleotide werden durch Chromatographie an einer kleinen Sephadex-G50-Säule abgetrennt.

c) Subklonieren des kompletten δ-Endotoxin Gens im pUC8-Vektor: des pK36 Klons

Der pUC8-Vektor (New England Biolabs) wird mit den Restriktionsenzymen HincII und PstI sowie durch Behandlung mit alkalischer Phosphatase (siehe Punkt III.1. oben) vollständig verdaut. Das HpaI/PstI-Fragment von pK25-7 (siehe Punkt III.6.a) oben), das für das δ-Endotoxin-Gen kodiert (Tabelle 2) wird mit der Vektor DNA verknüpft und in E.coli HB101 Zellen transformiert. Einer der korrekt transformierten Klone erhält die Bezeichnung pK36.

III.7. Biotest zur Bestimmung des B. thuringiensis Toxins:

Zu dem gereinigten Lysat, das entsprechend den unter Punkt III.3. gemachten Angaben erhalten werden kann, wird Ammoniumsulfat in einer 30 % Sättigungskonzentration hinzugegeben. Das Präzipitat

14

wird in 2 ml 50 mM Natriumcarbonat, pH 9.5, gelöst und gegen den gleichen Puffer dialysiert. Als Kontrolle werden E.coli Extrakte hergestellt, die zwar die Vektor DNA aber ohne eingebaute B. thuringiensis DNA enthalten. Die E.coli Zellextrakte werden zunächst mit Ultraschall behandelt, anschliessend werden 4 Konzentrationen entsprechend dem Toxin-Gehalt in den Extrakten hergestellt und mit 0,1 % (v/v) eines Benetzungsmittels vermischt.

Blattscheibchen von Baumwollpflanzen, die unter kontrollierten Bedingungen in einer Klimakammer (25°C, 60 % relative Luftfeuchtigkeit) herangezogen worden sind, werden in die E.coli Zellextrakt-Suspensionen eingetaucht. Heliothis virescens Larven im ersten Larvenstadium (30 Larven pro Konzentration), die zuvor in einem Fitnesstest standardisiert worden sind, werden dann auf die getrockneten Blattscheibchen gesetzt und einzeln für 3 Tage bei 25°C inkubiert.

Die Mortalität wird in % gemessen, wobei nur die Kriterien 'tot' oder 'lebendig' Anwendung finden. Die Extrakte, die Mortalität hervorrufen, besitzen daher bioinsektizide Aktivität, die von der klonierten B. thuringiensis DNA stammt.

## IV. DNA-Sequenzierung

Beide Stränge der DNA-Fragmente, die für das δ-Endotoxin-Gen kodieren, werden nach der Methode von F. Sanger et al.[24] unter Verwendung des M13 Systems (J. Messing[25]) sequenziert.

## IV.1. Klonieren des δ-Endotoxin-Gens in die replikative DNA-Form des M13

Aus der Restriktionskartierung des klonierten δ-Endotoxin-Gens und der Southern-Blot-Analyse lässt sich erkennen, dass das Gen auf zwei DNA Fragmenten von pK36 lokalisiert ist: HpaI (Position 0 auf der Sequenz) bis HindIII (Position 1847) und EcoRI (Position 1732) bis PstI (Position 4355). Das erste Fragment wird in M13mp8 (New England Biolabs) zwischen der einzigen HincII und der einzigen HindIII Stelle in einer Reaktion kloniert, die analog dem oben beschriebenen Verknüpfungs-Prozess abläuft.

## IV.2. Herstellung einer aufeinanderfolgenden Reihe überlappender Klone

Zur Verkürzung des HpaI-HindIII DNA Fragments, das für das 5'-Ende des Gens kodiert, kommt die Bal31 Methode (M.Poncz et al.[26]) zur Anwendung. Besagtes Verkürzen wird folgendermassen durchgeführt: Das Fragment wird in M13mp8 zwischen den einzigen HincII und HindIII-Stellen kloniert. 10 mg der replikativen DNA Form wird mit der Restriktionsendonuclease HindIII linearisiert und anschliessend mit der Endonuclease Bal31 in 100 $\mu$l 600 mM NaCl, 12 mM $CaCl_2$, 12 mM $MgCl_2$, 20 mM Tris•HCl, pH 8 und 1 mM EDTA, behandelt. Diese Mischung wird bei 30°C für 5 Minuten vorinkubiert. Anschliessend werden 5 Einheiten Bal31 zugegeben. Unmittelbar nach dieser Zugab sowie nach 2, 4, 6, 8, 10 und 12 Minuten werden jeweils 13 $\mu$l entnommen. Um hier eine weitere Reaktion zu verhindern werden gleich nach erfolgter Entnahme 25 $\mu$l Phenol und 40 $\mu$l TE-Puffer zugesetzt. Diese Mixtur wird zentrifugiert, mit Chloroform extrahiert und mit Ethanol prezipitiert. Das so erhaltene DNA Prezipitat wird in 20 $\mu$l 100 mM NaCl, 20 mM Tris•HCl und 10 mM $MgCl_2$ resuspendiert und mit einem zweiten Enzym verdaut, das auf der anderen Seite des ursprünglich klonierten Fragments gefunden wird; im vorliegenden Fall handelt es sich dabei um BamHI. Nach Auftrennung in einem Agarose-Gel entsprechend der Grösse, werden die verkürzten Fragmente mit Ethidiumbromid gefärbt und unter langwelligem UV-Licht bei 366 nm sichtbar gemacht.

Der Teil des Agarose-Gels, der die gekürzten Fragmente enthält, wird aus dem Gel herausgeschnitten, bei 65°C verflüssigt, auf 500 mM NaCl eingestellt und bei 65°C für 20 Minuten inkubiert. Ein Volumenteil Phenol (äquilibriert mit 10 mM Tris•HCl, pH 7.5, 1 mM EDTA, 500 mM NaCl) wird zugegeben.

Die wässrige Phase wird zweimal mit Phenol und einmal mit Chloroform reextrahiert. Die DNA wird mit 2,5 Volumenteilen kalten absoluten Ethanols prezipitiert und durch Zentrifugation gesammelt. Das DNA Pellet wird mit kaltem 80%igen Ethanol gewaschen und anschliessend im Vakuum getrocknet. Die DNA wird dann in 20 $\mu$l TE resuspendiert.

Die Fragmente werden successiv um 200-300 Bp verkürzt und besitzen auf der einen Seite des Fragments eine einzelne BamHI Restriktionsstelle und ein glattes Ende auf der anderen Seite. Diese Fragmente werden in einem M13mp8 Vektor kloniert, der zuvor durch zweifache Verdauung mit BamHI und HincII, wie oben unter Punkt IV.1 beschrieben, linearisiert wird.

Bei dem oben beschriebenen Verfahren wird die DNA Sequenz nur eines DNA Stranges erhalten, die bei der HindIII Restriktionsstelle beginnt und in Richtung der BamHI-Stelle fortschreitet.

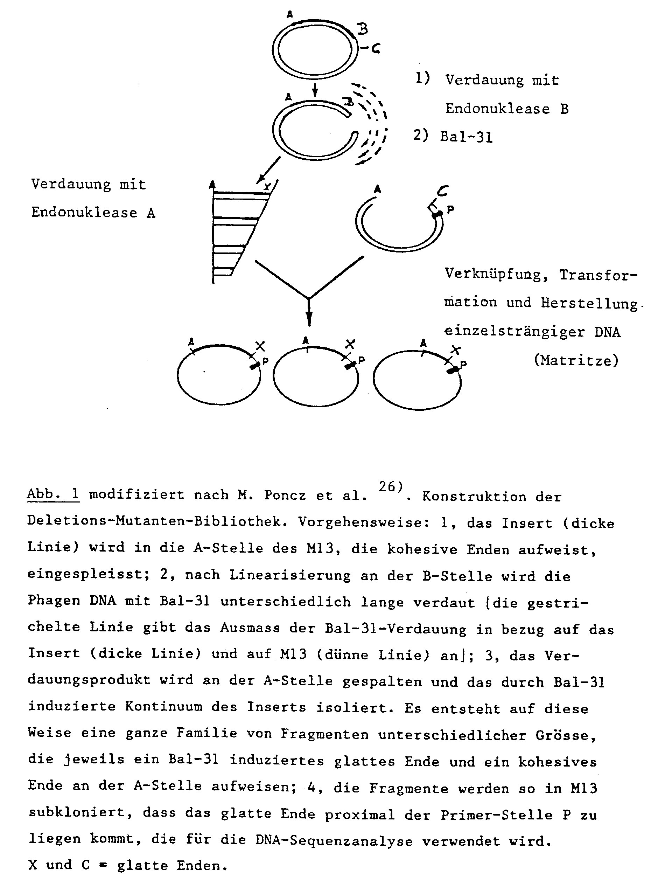

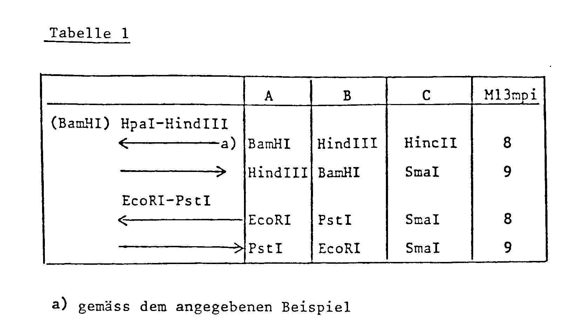

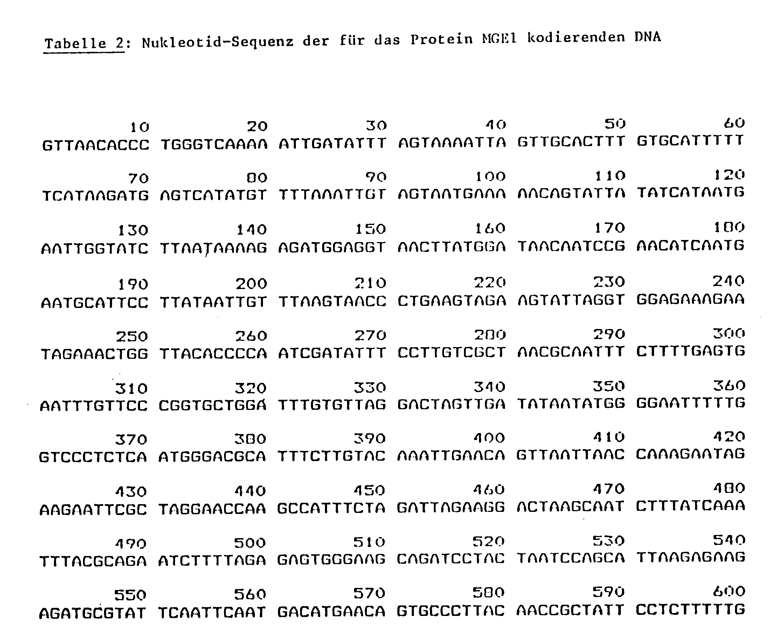

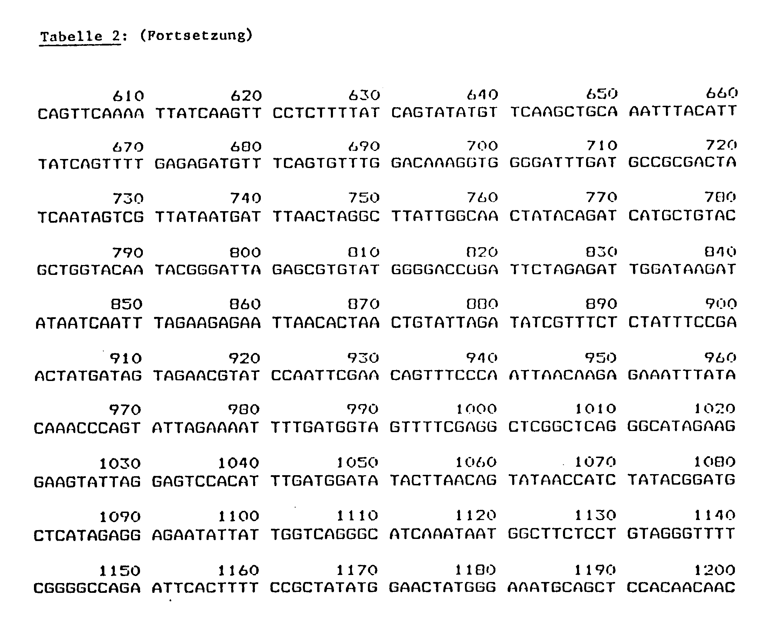

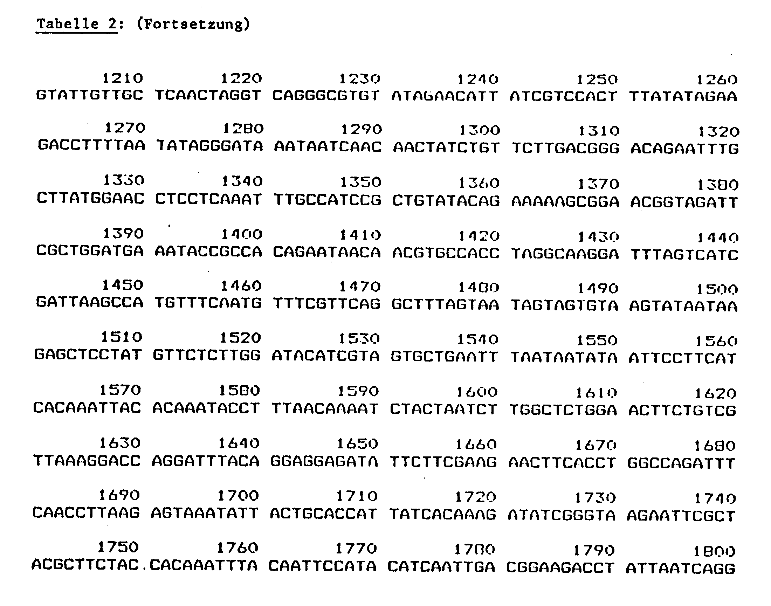

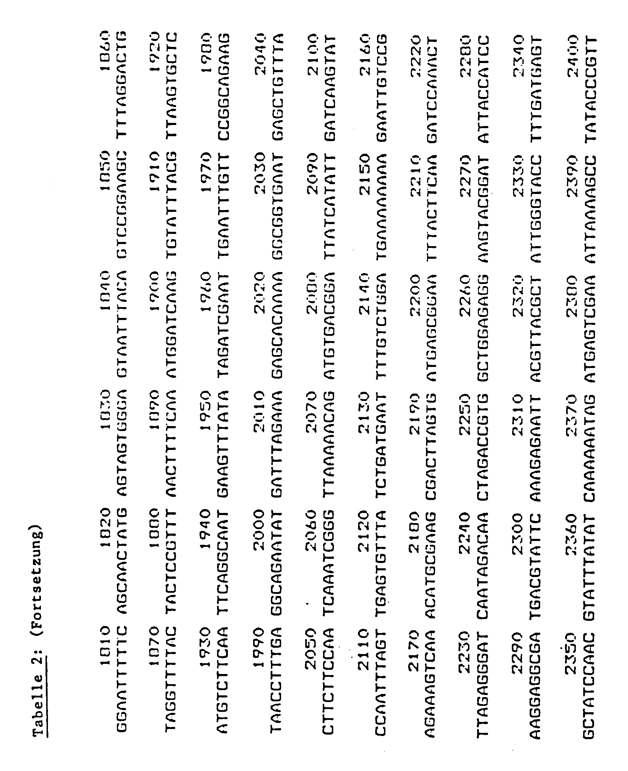

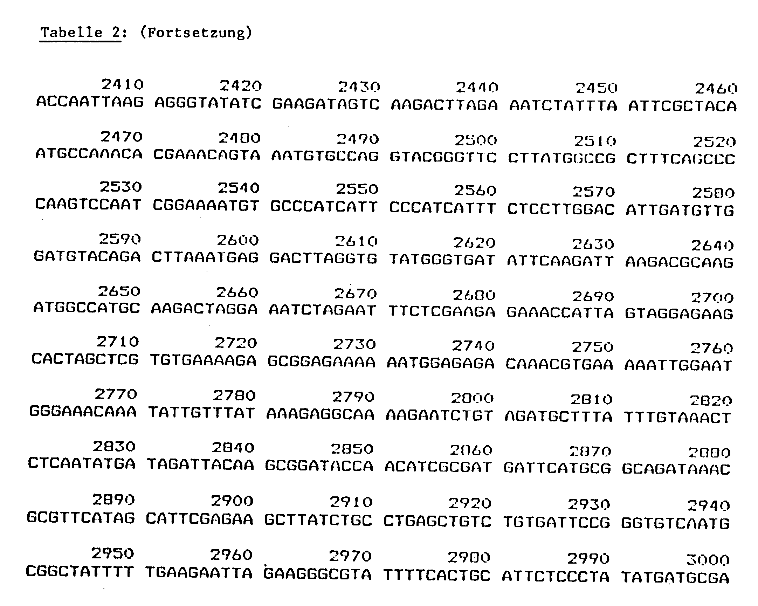

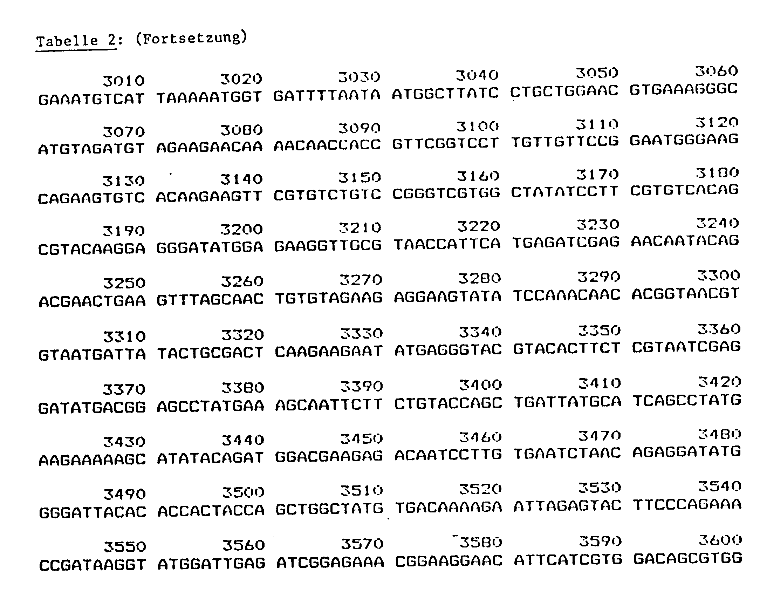

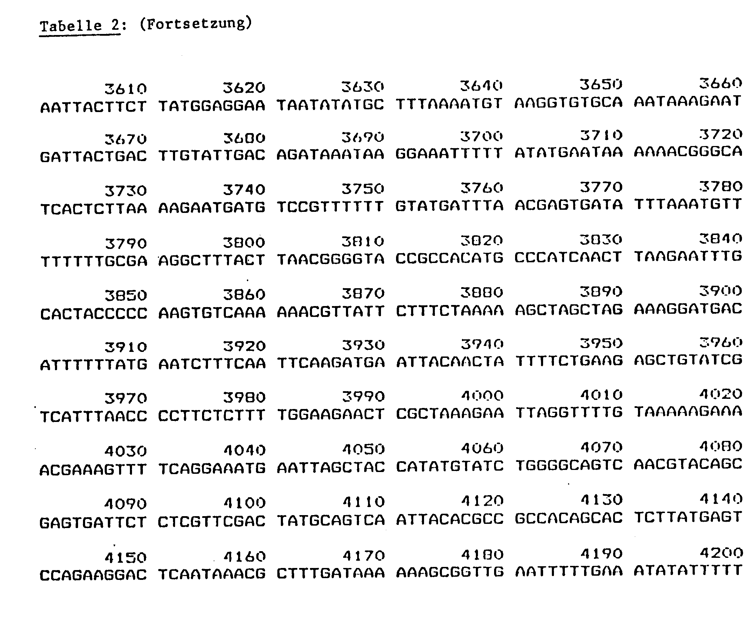

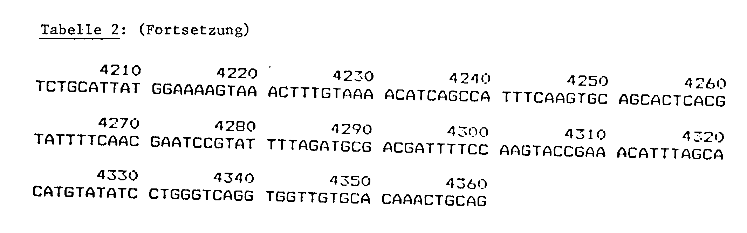

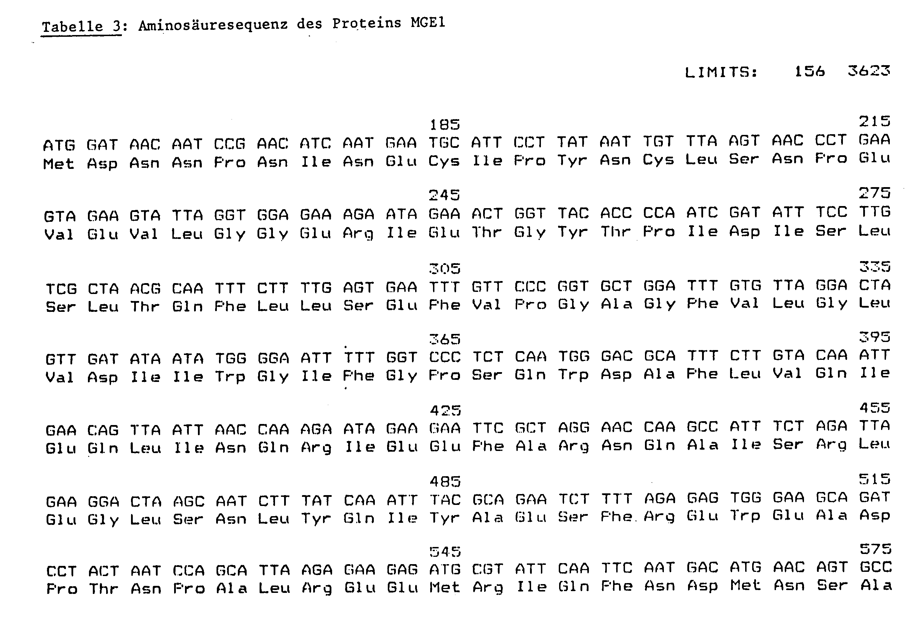

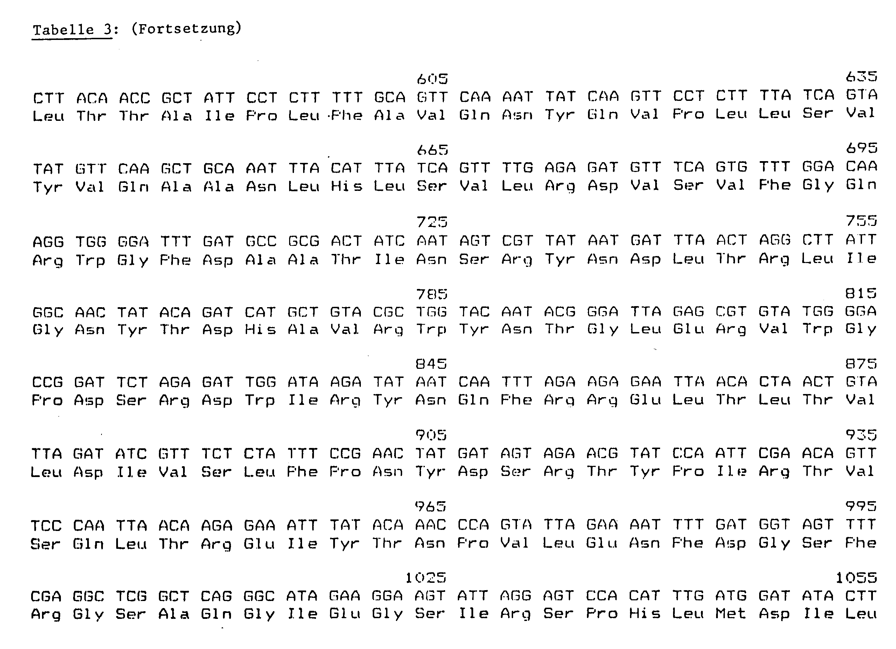

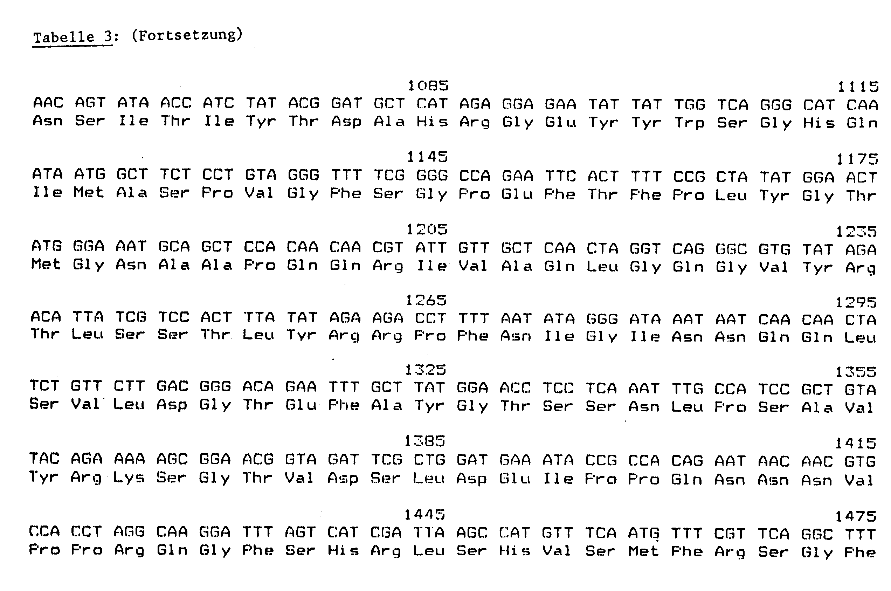

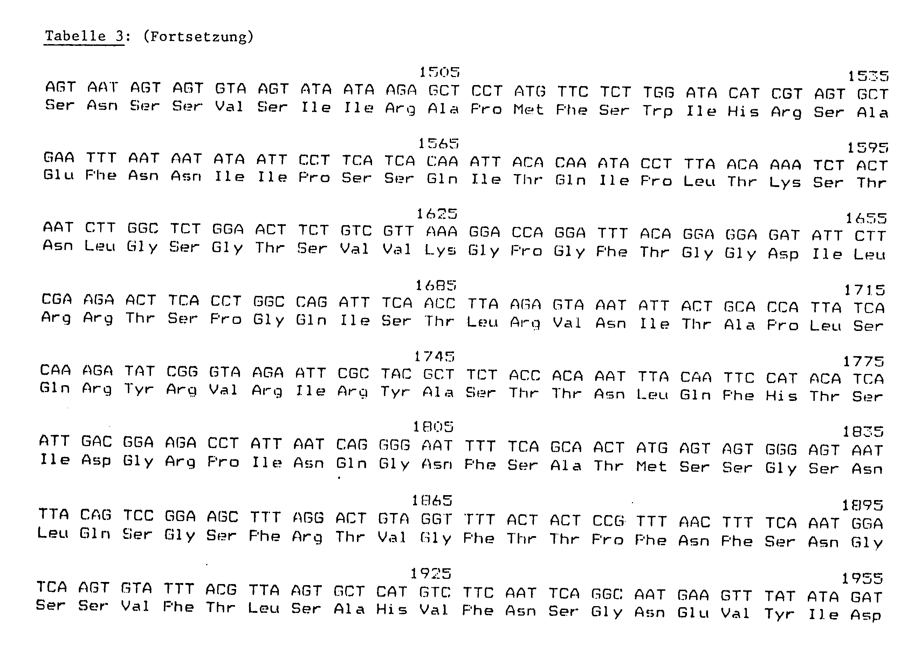

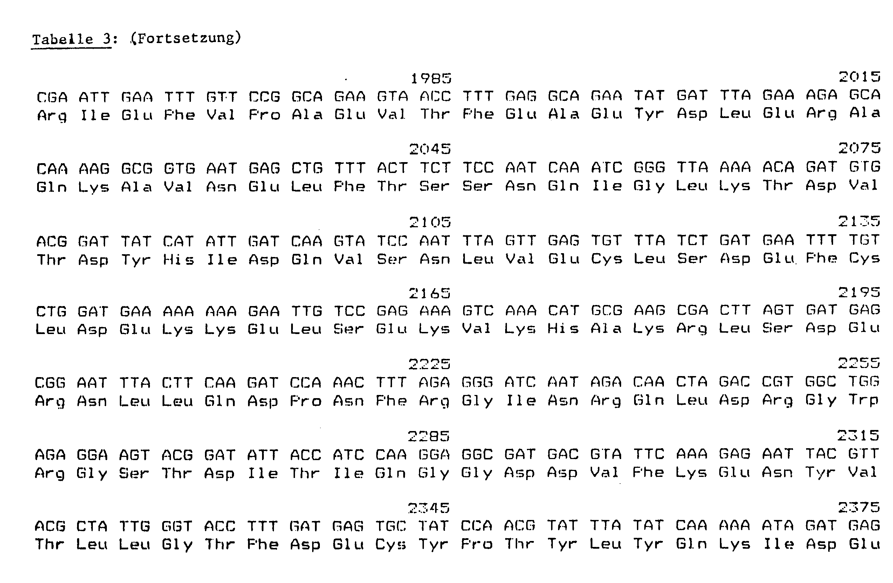

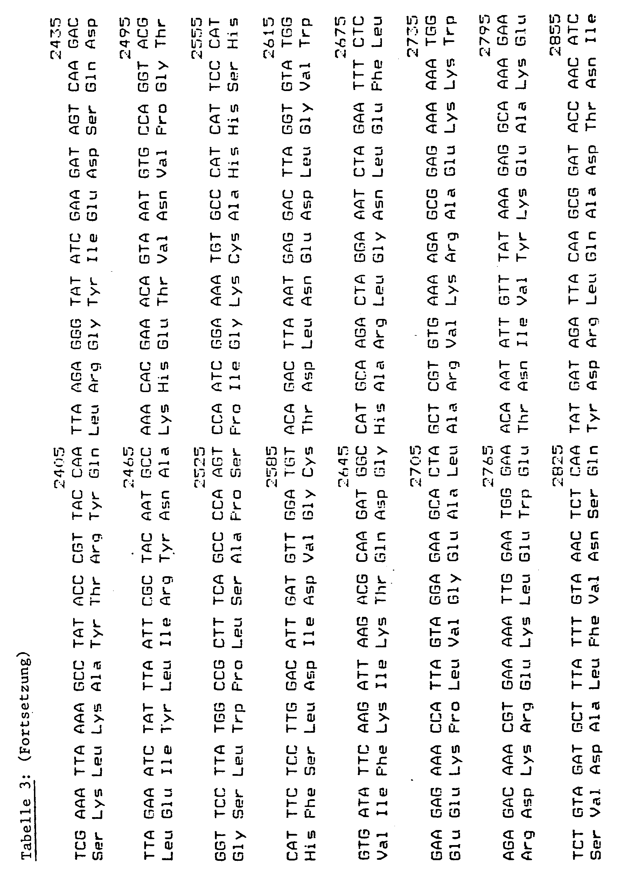

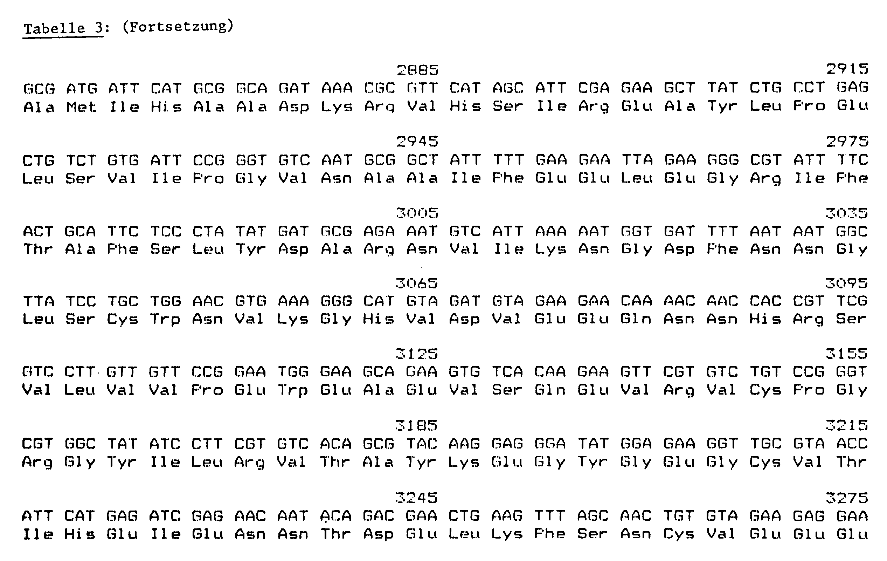

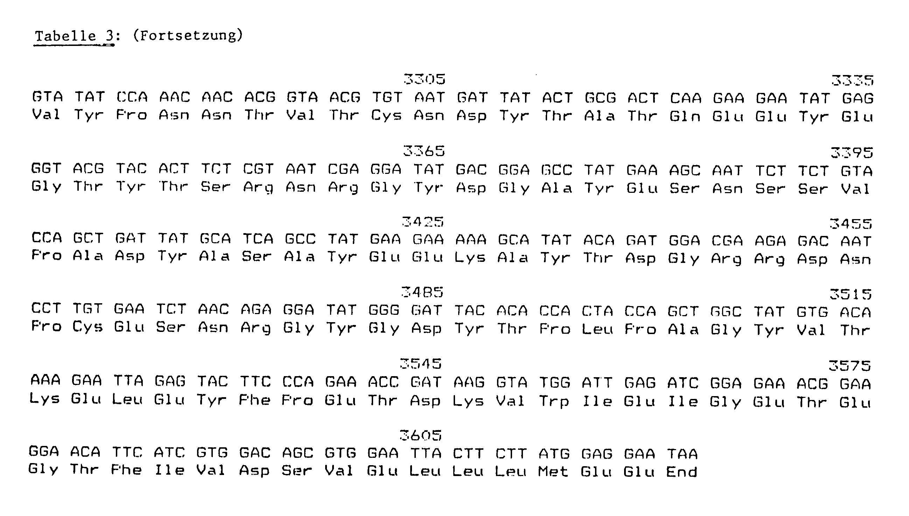

Die Vorgehensweise für Sequenzierung des komplementären DNA-Stranges desselben DNA-Fragments sowie die Restriktions-Schnittstellen der für die Sequenzierung verwendeten Endonucleasen, d.h. in erster Linie für die Verkürzung des zweiten DNA Fragments, weiterhin das EcoRI/Pst I-Fragment und dessen Sequenzierung sind in Abbildung 1, modifiziert nach Poncz et al. [26] und in Tabelle 1 wiedergegeben.

### IV.3. Transformation des E.coli Stammes JM103:

E.coli JM 103 Zellen werden auf M9 Maximal-Medium kultiviert. Die Transformation wird folgendermassen durchgeführt:

1. Eine einzelne E.coli JM 103 Kolonie in 2xYT inokulieren; über Nacht bei 37°C unter ständigem Rühren aufbewahren;

2. 40 ml 2xYT mit 200 $\mu$l der entsprechend Schritt 1 erhaltenen Kultur inokulieren;

3. Bei 37°C unter ständigem Rühren bis zu einer $OD_{550}$ von 0,5 kultivieren;

4. Auf Eis 5 Minuten inkubieren;

5. Bei 6000 rpm 5 Minuten in einem vorgekühlten SS34 Sorvall Rotor zentrifugieren;

6. Die Zellen in 20 ml einer 50 mM sterilen eisgekühlten $CaCl_2$-Lösung ($CaCl_2$-Lösung sollte jeweils frisch hergestellt werden) suspendieren;

7. Auf Eis 40 Minuten inkubieren;

8. Bei 6000 rpm 5 Minuten in einem SS34 Sorvall Rotor zentrifugieren;

9. Die Zellen in 3 ml einer eisgekühlten $CaCl_2$-Lösung suspendieren;

10. 1-5 $\mu$l DNA oder 7-15 $\mu$l einer Ligase Formulierung zu 200 $\mu$l der entsprechend Schritt 9 erhaltenen Zellen zugeben;

11. Auf Eis 30 Minuten inkubieren;

12. Bei einer Temperatur von 42°C, 3 min inkubieren;

13. 200 $\mu$l der entsprechend Schritt 1 erhaltenen Zellen zugeben;

14. Oberflächenagar aufkochen und bei 42°C aufbewahren;

15. Röhrchen mit 3 ml Oberflächenagar, 30 $\mu$l X-GAL (20 mg/ml Dimethylsulfoxid) und 30 $\mu$l IPTG (20 mg/ml $H_2O$) füllen;

16. Sorgfältig durchmischen und sofort in zuvor erwärmte 1xYT Platten überführen;

17. Platten für ca 1 Stunde trocknen lassen;

18. Platten umdrehen und bei 37°C inkubieren.

Die auf diese Weise erhaltenen Plaques sind für die weitere Behandlung geeignet.

```
Kontrollen: -   200 µl kompetente Zellen ohne exogene DNA

            +   1 µl Ml3mp8 (replikative DNA-Form, 10 ng) + 200 µl
                kompetenter Zellen
```

### IV.4. Herstellung der replikativen Form einer rekombinanten Phagen DNA

1. Einzelne weisse Plaques vorsichtig in 9 ml 2xYT und 1 ml der nach Schritt 1 Teil IV.3 erhaltenen Kultur überführen und 7 Stunden bei 37°C inkubieren

2. Bei 4000 rpm 10 Minuten zentrifugieren;

3. Ueberstand über Nacht bei 4°C aufbewahren;

4. 10 ml des entsprechend Schritt 3 erhaltenen Ueberstandes sowie von 10 ml der entsprechend Schritt 1, Teil IV.3 erhaltenen Kultur in 1 l 2xYT inokulieren;

5. Bei 37°C 4 1/2 Stunden schütteln;

6. Bei 5000 rpm 15 min zentrifugieren;

7. Die Zellen in 10 ml einer 10 % Sucroselösung in 50 mM Tris•HCl, pH 8 suspendieren und abkühlen;

8. In 30 ml-Zentrifugenröhrchen überführen;

9. 2 ml frisch hergestellte Lysozym-Lösung (10 mg/ml 0,25 M Tris•HCl, pH 8) hinzugeben;

10. 8 ml 0,25 M EDTA zugeben und vorsichtig vermischen;

11. 10 min auf Eis inkubieren;

12. 4 ml 10 % SDS (oder 1,6 ml 25 % SDS) zugeben und mit einem Glasstab vermischen;

13. 6 ml 5 M NaCl (Endkonzentration: 1 M) zugeben und vorsichtig vermischen;

14. 1 Stunde auf Eis inkubieren;

15. 40 min bei 20'000 rpm in einem SS34 Sorvall Rotor zentrifugieren;

16. Ueberstand entnehmen und 1/10 Volumen 5 M NaCl und 15 ml 30 % PEG in TNE zugeben;

17. 2 Stunden oder über Nacht bei 4°C inkubieren;

18. Bei 8'000 rpm 15 Minuten zentrifugieren;

19. Pellets entnehmen und in 18 ml TE, pH 8 überführen;

20. 18 g CsCl (1 g/ml) zugeben;

21. Entweder in Ti-50-Röhrchen überführen und 0,4 ml Ethidiumbromid (10 mg/ml) zugeben oder in Ti-60-Röhrchen überführen unter Zugabe von 1,2 ml Ethidiumbromid (10 mg/ml).

22. Mit CsCl-Lösung (1 g CsCl + 1 ml TE) auffüllen;

23. Bei 35'000 rpm und 20°C 36-48 Stunden zentrifugieren;

24. Die unter UV-Licht (366 nm) sichtbaren unteren Banden entnehmen;

25. Mit mit Wasser gesättigtem Butanol 3-4 mal extrahieren;

26. Bei 4°C 3 mal jeweils gegen 1 l steriles TE dialysieren;

IV.5. Herstellen einer Einzelstrang DNA Matrize:

1. E.coli-JM 103-Zellen in 5 ml 2xYT-Medium bei 37°C über Nacht schütteln;

2. 2 Tropfen einer entsprechend Schritt 1 erhaltenen Zellsuspension zu 25 ml 2xYT Medium zugeben;

3. Zwei Röhrchen mit jeweils 2 ml der gemäss Schritt 2 erhaltenen Kultur-Lösung füllen und 1 Plaque pro Röhrchen, das entsprechend den Angaben unter Punkt IV.3. oben erhalten wird, zugeben;

4. 5 1/2 Stunden bei 37°C schütteln;

5. Röhrcheninhalt in Eppendorf-Röhrchen überführen und 5 Minuten zentrifugieren;

6. 1 ml des Ueberstandes in frische Eppendorf-Röhrchen überführen;

7. 200 $\mu$l 20 % PEG 6000/2,5 M NaCl zugeben;

8. 15 Minuten bei RT inkubieren;

9. 5 Minuten zentrifugieren;

10. Ueberstand abheben und erneut kurzzeitig zentrifugieren;

11. Ueberstand vorsichtig abheben durch Ansaugen mit einer in die Länge gezogenen Pasteurpipette;

12. Zu dem verbleibenden Rest 100 $\mu$l TE und 50 $\mu$l Phenol zugeben;

13. 10 Sekunden mischen (mit dem Vortex); 5 min stehen lassen; 10 sec mischen (mit dem Vortex); 1 min zentrifugieren;

14. Wässrige Phase in frische Eppendorf-Röhrchen überführen;

15. 500 $\mu$l Ethylenether zugeben, mischen (Vortex) und 1 min zentrifugieren;

16. Ether durch Ansaugen entfernen und Röhrchen für 10 Minuten unverschlossen lassen (falls die wässrige Phase nach dieser Behandlung sehr trüb ist, sollte mit der Pasteurpipette Luft durchgeblasen werden, bis die Lösung klar ist);

17. 10 $\mu$l 3 M Natriumacetat und 250 $\mu$l Ethanol zugeben;

18. 30 Minuten bei -80°C inkubieren;

19. 5 Minuten zentrifugieren;

20. Mit 80 % Ethanol waschen;

21. 5 Minuten zentrifugieren;

22. Ueberstand mit verlängerter Pasteur-Pipette abheben;

23. Röhrchen 15 Minuten unverschlossen lassen;

24. Pellet in 25 $\mu$l TE lösen;

25. 2 $\mu$l der Pellet-Lösung auf ein 0,6 % Agarose-Gel auftragen;

IV.6. Sequenzierungsreaktion

Die DNA Sequenzanalyse der Matrizen-DNA, die entsprechend den Angaben unter Punkt IV.5. erhalten werden kann, wird nach der im Handbuch "M13 Klonierungs- und DNA Sequenzierungs-System", publiziert bei New England Biolabs, beschriebenen Methode durchgeführt.

Die Analyse der kompletten DNA-Sequenz zeigt, dass man lediglich einen offenen Leserahmen findet, der genügend lang ist für ein Protein mit einem MG von 130.622 und der für 1155 Aminosäuren kodiert.

Der N-Terminus des Proteins befindet sich 156 Bp stromabwärts der HpaI-Restriktionsstelle, die letze Aminosäure des C-Terminus dagegen wird durch ein Kodon kodiert, das bei Nukleotid 3618 beginnt. Die DNA-Sequenz zwischen der HpaI und der PstI Schnittstelle ist in Tabelle 2, die daraus abgeleitete Aminosäuresequenz in Tabelle 3 wiedergegeben.

## V. Expression des Endotoxin-Gens in Hefe-Zellen

### V.1. Einführen einer NcoI Schnittstelle vor dem ersten AUG-Kodon des Gens

Um die Protein-kodierende Sequenz des B. thuringiensis Toxin-Gens mit dem PH05 Hefe-Promotor kombinieren zu können (beschrieben in der Europäischen Patentanmeldung Nr. 100,561), wird die DNA-Sequenz in der Umgebung des Toxin Gens modifiziert. Diese Modifikation wird durch Oligonucleotid-vermittelte Mutagenese mit dem einzelsträngigen Phagen-Vektor M13mp8 erreicht, der ein 1,5 Kb BamHI-SacI Insert besitzt, das für die 5'-Region des Toxin-Gens kodiert. 200 ng des Inserts werden durch Verdauung von 3 $\mu$g Plasmid DNA des Plasmids pK36 mit BamHI und SacI und durch anschliessende Isolierung des Fragments unter Verwendung von oben beschriebenen Standardmethoden erhalten. 100 ng der replikativen Form (RF) von M13mp8 werden mit den gleichen Enzymen verdaut, die DNA wird mit Phenol behandelt und durch Ethanol-Zugabe prezipitiert und anschliessend mit 200 ng der oben erwähnten Insert-DNA verknüpft. Nach Transfektion von E.coli werden sechs weisse Plaques herausgegriffen und durch Restriktionsverdauungen unter Verwendung von BamHI und SacI analysiert. Ein korrektes Isolat wird ausgewählt und als M13mp18/Bam-Sac bezeichnet.

Ein Oligonukleotid mit der Sequenz (5') GAGGTAACCCATGGATAAC (3') wird mit Hilfe an sich bekannter Methoden unter Verwendung eines 'APPLIED BIOSYSTEMS DNA SYNTHESIZER' synthetisiert. Diese Oligonucleotid ist komplementär zu einer Sequenz der M13mp18/Bam-Sac, die von Position 141 bis Position 164 des Protoxin Gens reicht (Tabelle 2) und die in den Postionen 154 und 155 falsch gepaarte Nukleotidpaare ('Mismatch') aufweisen. Die allgemeine Vorgehensweise bei der Mutagenese ist bei J.M. Zoller und M. Smith (J.M. Zoller and M. Smith[27]) beschrieben. Etwa 5 $\mu$g einzelsträngiger M13mp18/Bam-Sac-Phagen-DNA wird mit 0,3 $\mu$g phosphorylierten Oligonukleotiden in einem Gesamt-Volumen von 40 $\mu$l gemischt. Diese Mischung wird für 5 Minuten auf 65°C erhitzt, dann zunächst auf 50°C abgekühlt und anschliessend allmählich auf 4°C heruntergekühlt. Danach werden Puffer, Nucleotidtriphosphate, ATP, T4-DNA-Ligase und das grosse Fragment der DNA-Polymerase hinzugefügt und über Nacht bei 15°C, wie beschrieben (J.M. Zoller and M. Smith[27]) inkubiert. Nach einer Agarose-Gel-Elektrophorese wird zirkuläre doppelsträngige DNA gereinigt und mittels Transfektion in den E.coli Stamm JM103 eingeschleust.

Die resultierenden Plaques werden auf Sequenzen hin untersucht, die mit $^{32}$P-markiertem Oligonukleotid hybridisieren; die Phagen werden mit Hilfe der DNA Restriktionsendonukleasen-Analyse untersucht. Unter den resultierenden Phagen werden die als Klone bezeichnet, die jetzt korrekterweise an Stelle von T in der pK36 DNA ein C an Position 154 und 155 aufweisen. Diese Phagen werden als M13mp18/Bam-Sac/Nco bezeichnet.

### V. 2. Verknüpfung des δ-Endotoxin-Gens mit dem PH05-Hefe-Promotor

Das 1.5 Kb BamHI-SacI Insert des M13mp18/Bam-Sac/Nco wird in das Plasmid pK36 zurückkloniert, indem das Wildtyp BamHI-SacI-Fragment von pK36 durch das mutierte 1,5 Kb Fragment unter Verwendung von zuvor beschriebenen Standard-Klonierungs Techniken ersetzt wird.

Dadurch entsteht das Plasmid pK36/Nco, das eine NcoI Restriktionsstelle unmittelbar vor dem ATG des Protoxin-Gens aufweist

```
              Noc I
....GAGGTAAC/CCATGG/ATAAC.
```

5 $\mu$g dieses Vektors wird mit NcoI verdaut und die überhängenden 3' Enden werden mit Klenow Polymerase aufgefüllt, wie bei Maniatis et al.[28] beschrieben. Anschliessend wird das Plasmid mit AhaIII verdaut, die DNA auf einem 0,8%igen niedrig schmelzenden Agarose-Gel aufgetrennt und wie oben beschrieben eluiert. 2 $\mu$g des Plasmids p31y (beschrieben in der Europäischen Patentanmeldung Nr. 100,561) wird mit EcoRI verdaut und die zurückgesetzten Enden werden wie zuvor beschrieben mit Klenow Polymerase aufgefüllt. Die Verknüpfung des stumpf endenden 3.6 Kb Protoxin Genfragments mit dem stumpf endenden Vektor p31y erfolgt durch Inkubation von je 200 ng beider DNA's in 20 $\mu$l bei RT wie bei Maniatis et al.[29] beschrieben. Nach Transformation einer Ampicillin Resistenz auf E.coli HB101 werden einzelne Klone einer Restriktionsanalyse unterzogen. Ein korrektes Isolat wird herausgesucht und mit der Bezeichnung p31y/B.t. versehen. 1 $\mu$g dieser Plasmid DNA wird mit BamHI verdaut und das 4 Kb Fragment aus einem weichen Agarose Gel isoliert. Dieses Fragment wird mit dem selbst-replizierenden Hefe-Vektor pJDB207 (beschrieben in der Europäischen Patentanmeldung Nr. 100,561) verknüpft, der zuvor ebenfalls mit BamHI (0,5 $\mu$g) verdaut worden ist. Positive Klone werden mit Hilfe der E.coli Transformation und der Plasmid DNA Aufarbeitung isoliert. Korrekte Isolate lassen sich anhand einer Restriktionsanalyse unter Verwendung von BamHI ermitteln.

Die Transformation des Hefe Stammes GPF18 /(MATα, leu 2-3, leu 2-112, his 3-11, his 3-15 can.[R]) wird entsprechend den Angaben in der Europäischen Patentanmeldung 100,561 durchgeführt.

### V.3. Ganze Hefe-Zellen mit einem rekombinanten B. thuringiensis Toxin-Gen im Biotest

Ganze Zellen mit dem B. thuringiensis Gen stellen eine Bioenkapsulierungs-Form (ein artifiziell erstelltes System, welches aus biologischem Material besteht, und wobei genetisches Material von schützendem Material umgeben ist) für das MGE1-Produkt dar, das jetzt beim Aufbringen auf die Pflanzen besser gegen Abbau durch schädliche Einflüsse, wie z.B. Licht, geschützt ist, als das kristalline Produkt, das von B. thuringiensis im Rahmen der Sporulation gebildet wird und durch Aufbrechen der Zelle ins Medium gelangt.

Hefe-Zellen mit und ohne dem B. thuringiensis Toxin werden in destilliertem Wasser bis zu einer vergleichbaren optischen Dichte resuspendiert. Von besagter Suspension werden 4 Konzentrationen bereitgestellt und 0,2 % (v/v) eines Benetzungsmittels beigemischt. Für die Beurteilung der insektiziden Aktivität dieser Hefezell-Präparate wird der oben unter Punkt III.7 bereits beschriebene Blattscheiben-Test herangezogen.

Die insektizide Aktivität von B. thuringiensis transformierten Hefe Zellen auf im ersten Larvenstadium befindliche Heliothis virescens Larven wird in der folgenden Tabelle demonstriert.

### Tabelle 4

| Material | Konzentration | Mortalität in % (N = 30) |
|---|---|---|
| Zellen mit Toxin | 1:5 | 72 |
| | 1:7,5 | 40 |
| | 1:11,3 | 37 |
| | 1:16,9 | 22 |
| Zellen ohne Toxin | 1:5 | 3 |
| | 1:11,3 | 0 |
| Blattstückchen ohne Hefe: | | |
| Kontrolle 1 | -- | 0 |
| Kontrolle 2 | -- | 3 |

Aehnliche Ergebnisse sind erhältlich, wenn man Hefeextrakte, die entsprechend der Beschreibung in der Europäischen Patentanmeldung 100,561 hergestellt worden sind, anstelle von ganzen Hefezellen verwendet und diese im gleichen Biotest testet.

Für die Anwendung als Insektizide werden die transformierten Mikroorganismen, die das rekombinante B. thuringiensis Toxin-Gen enthalten, vorzugsweise transformierte lebende oder tote Hefe-Zellen, einschliesslich Gemischen aus lebenden und toten Hefe-Zellen, in unveränderter Form oder vorzugsweise zusammen mit den in der Formulierungstechnik üblichen Hilfsstoffen, eingesetzt und werden daher in an sich bekannter Weise formuliert, z.B. zu Suspensionskonzentraten, streichbaren Pasten, direkt versprühbaren oder verdünnbaren Lösungen, benetzbaren Pulvern, löslichen Pulvern, Stäubemitteln, Granulaten, und auch Verkapselungen in z.B. polymeren Stoffen.

Die Amwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt.

Die Formulierungen, d.h. die die transformierten lebenden oder toten Hefe-Zellen oder Mischungen davon und gegebenenfalls feste oder flüssige Hilfsmittel enthaltenden Mittel oder Zubereitungen werden in bekannter Weise hergestellt, z.B. durch inniges Vermischen der Hefezellen mit festen Trägerstoffen und gegebenenfalls oberflächenaktiven Verbindungen (Tensiden).

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen nichtionogene, kation- und/oder anionaktive Tenside mit guten Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder unsubstituierte oder substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-taurinsalze zu erwähnen, wie z.B. das Natriumsalz der cis-2-(methyl-9-octadecenylamino)-ethansulfonsäure (Gehalt in Formulierungen vorzugsweise etwa 3 %).

Häufiger werden jedoch sogenannte synthetische Tenside verwendet, insbesondere Fettsulfonate, Fettsulfate, sulfonierte Benzimidazolderivate oder Alkylarylsulfonate oder Fett-Alkohole, wie z.B. 2,4,7,9-tetramethyl-5-decin-4,7-diol (Gehalt in Formulierungen vorzugsweise bei etwa 2 %).

Die Fettsulfonate oder -sulfate liegen in der Regel als Alkali-, Erdalkali- oder gegebenenfalls unsubstituierte oder substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylsulfats oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4 bis 14)-ethylenoxid-Adduktes in Frage.

Als nicht-ionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nicht-ionische Tenside sind die wasserlöslichen, 20 bis 250 Ethylenglykolethergruppen und 10 bis 100 Propylenglykolethergruppen enthaltenden Polyethylenoxid-Addukte an Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nicht-ionischer Tenside seien Nonylphenolpolyethoxyethanole, Ricinusölpolyglykolether, Polypropylen/Polyethylenoxid-Addukte, Tributylphenoxypolyethoxyethanol, Polyethylenglykol und Octylphenoxypolyethoxyethanol erwähnt. Ferner kommen auch Fettsäureester von Polyoxyethylensorbitan wie das Polyoxyethylensorbitantrioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten unsubstituierte oder halogenierte Nieder-Alkyl-, -Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)-ethylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ridgewood New Jersey, 1980;

Helmut Stache "Tensid-Taschenbuch" Carl Hanser-Verlag München/Wien 1981.

Die agrochemischen Mittel enthalten in der Regel 0,1 bis 99 %, insbesondere 0,1 bis 95 %, der transformierten, lebenden oder toten Hefezellen oder Mischungen davon, 99,9 bis 1 %, insbesondere 99,8 bis 5 %, eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Formulierungen.

Solche Mittel können noch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die transformierten lebenden oder toten Hefe-Zellen oder Mischungen davon, die die rekombinanten B. thuringiensis Toxin-Gene enthalten, sind hervorragend für die Bekämpfung von Schadinsekten geeignet. Vorzugsweise sind dabei pflanzenzerstörende Insekten der Ordnung Lepidoptera zu nennen, insbesondere solche der Gattungen Pieris, Heliothis, Spodoptera und Plutella, wie beispielsweise Pieris brassicae, Heliothis virescens, Heliothis zea, Spodoptera littoralis und Plutella xylostella.

Die Aufwandmengen, in denen die Hefezellen eingesetzt werden, hängen von den jeweiligen Bedingungen ab, wie beispielsweise den Witterungsverhältnissen, der Bodenbeschaffenheit, dem Pflanzenwachstum und dem Applikationszeitpunkt. Aufgrund von Vorversuchen, die im Gewächshaus durchgeführt wurden, kann davon ausgegangen werden, dass Auffwandmengen von 1 bis 10 kg, insbesondere 3 bis 9 kg, der Hefe-Zellen pro Hektar vorteilhaft sind.

Formulierungsbeispiele für B. thuringiensis-Toxin enthaltendes Material

Bei den folgenden Formulierungsbeispielen sind unter dem Begriff "Hefe-Zellen" solche zu verstehen, die das rekombinante B. thuringiensis-Gen enthalten. (Bei den Angaben handelt es sich durchgehend um Gewichstprozente.)

| F1. Granulate | | |
|---|---|---|
| | a) | b) |
| Hefe-Zellen | 5 % | 10 % |
| Kaolin | 94 % | - |
| Hochdisperse Kieselsäure | 1 % | - |
| Attapulgit | - | 90 % |

Die Hefe-Zellen werden zunächst in Methylenchlorid suspendiert, anschliessend wird die Suspension auf das Trägermaterial aufgesprüht und danach das Suspendierungsagens im Vakuum verdampft.

| F2. Stäubemittel | | |
|---|---|---|
| | a) | b) |
| Hefe-Zellen | 2 % | 5 % |
| Hochdisperse Kieselsäure | 1 % | 5 % |
| Talkum | 97 % | - |
| Kaolin | - | 90 % |

Gebrauchsfertige Stäubemittel erhält man durch inniges Vermischen der Trägerstoffe mit den Hefe-Zellen.

| F3. Spritzpulver | | | |
|---|---|---|---|
| | a) | b) | c) |
| Hefe-Zellen | 25 % | 50 % | 75 % |
| Natrium-Ligninsulfonat | 5 % | 5 % | - |
| Natrium-Laurylsulfat | 3 % | - | 5 % |
| Natrium-Diisopropylnaphthalinsulfonat | - | 6 % | 10 % |
| Octylphenolpolyethylenglykolether (7-8 Mole Ethylenoxid | - | 2 % | - |
| Hochdisperse Kieselsäure | 5 % | 10 % | 10 % |
| Kaolin | 62 % | 27 % | - |

Die Hefezellen werden sorgfältig mit den Zusatzstoffen vermischt und das erhaltene Gemisch anschliessend in einer geeigneten Mühle gut vermahlen.

Man erhält Spritzpulver, die sich mit Wasser zu Suspensionen jeder gewünschten Konzentration verdünnen lassen.

| F4. Extruder Granulate | |
|---|---|
| Hefe-Zellen | 10 % |
| Natrium-Ligninsulfonat | 2 % |
| Carboxymethylcellulose | 1 % |
| Kaolin | 87 % |

Die Hefe-Zellen werden mit den Hilfsstoffen gemischt, sorgfältig vermahlen und mit Wasser angefeuchtet. Dieses Gemisch wird extrudiert und anschliessend in Luftstrom getrocknet.

| F5. Umhüllungs-Granulat | |
|---|---|
| Hefe-Zellen | 3 % |
| Polyethylenglykol 200 | 3 % |
| Kaolin | 94 % |

Die homogen vermischten Hefe-Zellen werden in einem Mischer auf das mit Polyethylenglykol angefeuchtete Kaolin gleichmässig aufgetragen. Auf diese Weise erhält man staubfreie Umhüllungs-Granulate.

| F6. Suspensions-Konzentrat | |
|---|---|
| Hefe-Zellen | 40 % |
| Ethylenglykol | 10 % |
| Nonylphenolpolyethylenglykol (15 Mole Ethylenoxid) | 6 % |
| Alkylbenzolsulfonsäuretriethanolaminsalz* | 3 % |
| Carboxymethylcellulose | 1 % |
| Silikonöl in Form einer 75 %igen wässrigen Emulsion | 0,1 % |
| Wasser | 39 % |

\* Alkyl ist vorzugsweise linear mit 10 bis 14, insbesondere 12-14 Kohlenstoffatomen wie beispielsweise n-Dodecylbenzolsulfonsäuretriethanolaminsalz.

Die homogen vermischten Hefe-Zellen werden mit den Zusatzstoffen innig vermischt. Man erhält so ein Suspensionkonzentrat, aus welchem durch Verdünnen mit Wasser Suspensionen jeder gewünschten Konzentration hergestellt werden können.

Von jedem der im folgenden aufgelisteten Mikroorganismen, die im Rahmen der vorliegenden Erfindung verwendet werden, wurde eine Kultur bei der als internationale Hinterlegungsstelle anerkannten 'Deutschen Sammlung von Mikroorganismen' in Göttingen, Bundesrepublik Deutschland, entsprechen den Anforderungen des Budapester Vertrages für die Internationale Anerkennung der Hinterlegung von Mikroorganismen zum Zwecke der Patentierung, hinterlegt. Eine Erklärung zur Lebensfähigkeit der hinterlegten Proben wurde durch die besagte Internationale Hinterlegungsstelle ausgefertigt.

| Mikroorganismen | Hinterlegungsdatum | Hinterlegungs-Nummer* | Datum der Lebensfähigkeitsbescheinigung |
|---|---|---|---|
| HD1-ETHZ 4449 (Bacillus thuringiensis var. kurstaki HD1 Stamm ETHZ 4449) | 4. März 1986 | DSM 3667 | 7. März 1986 |
| HB101 (pK36) (Escherichia coli HB101 transformiert mit pK36 Plasmid-DNA) | 4. März 1986 | DSM 3668 | 7. März 1986 |
| GRF 18 (Saccharomyces cerevisiae) | 4. März 1986 | DSM 3665 | 7. März 1986 |

* Eingangs-Nummer, ausgegeben durch die oben bezeichnete Internationale Hinterlegungsstelle.

Einschränkungen der Zugänglichkeit besagter Mikroorganismen sind seitens des Hinterlegers nicht verlangt worden.

Literatur:

[1] S. Chang, Trends in Biotechnology 1 (4), 100 (1983)

[2] H.C. Wong, H.E. Schnepf and H.R. Whiteley, The Journal of Biological Chemistry 258 (3), 1960 (1983)

[3] M.J. Adang, M.J. Staver, T.A. Rocheteau, J. Leighton, R.F. Barker and D.V. Thompson, Gene 36, 289 (1985)

[4] H.E. Schnepf, H.C. Wong and H.R. Whiteley, The Journal of Biological Chemistry 260 (10), 6264 (1985)

[5] A.A. Yousten and M.H. Rogoff, Journal of Bacteriology 100, 1229 (1969)

[6] T. Maniatis, E.F. Fritsch and J. Sambrook, Molekular Cloning: A Laboratory Manual, Cold Spring Harbor Laboratory, Cold Spring Harbor, USA, Appendix C: Commonly used bacterial strains, pp. 504, 506 (1982)

[7] F.F. White and E.W. Nester, Journal of Bacteriology 141 (3), 1134 (March 1980)

[8] B. Trümpi, Zentralblatt f. Bakteriologie Abt. II, 305 (1976)

[9] F.P. Delafield, H.J. Sommerville and S.C. Rittenberg, Journal of Bacteriology 96, 713 (1968)

[10] G.G. Chestukhina, I.A. Zalunin, L.I. Kostina, T.S. Kotova, S.P. Katrukha, L.A. Lyublinskaja and V.M. Stepanov, Biokhimija 43, 857 (1978)

[11] O. Ouchterlony, Handbook of Immunodiffusion and Immunoelectrophoresis, Ann Arbor Science Publishers, Ann Arbor, Mich., USA (1968)

[12] H. Huber-Lukac, Doktorarbeit, Eidgenössisch Technische Hochschule, Zürich, Schweiz, No. 7050 (1982)

[13] Amersham Buchler Review No. 18, Amersham Buchler GmbH & Co. KG, Braunschweig, Bundesrepublik Deutschland (1979)

[14] T. Maniatis, E.F. Fritsch and J. Sambrook, Molecular Cloning: A Laboratory Manual, Cold Spring Harbor Laboratory, Cold Spring Harbor, USA, p. 282 (1982)

[15] T. Maniatis, E.F. Fritsch and J. Sambrook, Molecular Cloning: A Laboratory Manual, Cold Spring Harbor Laboratory, Cold Spring Harbor, USA, p. 252 (1982)

[16] H.E. Schnepf and H.R. Whiteley, Proc.Natl.Acad.Sci. USA 78, 2893 (1981)

[17] L. Clarke, R. Hitzeman and J. Carbon, Methods in Enzymology 68, 436 (1979)

[18] E.M. Southern, J. Molec.Biol. 98, 503 (1975)

[19] T. Maniatis, E.F. Fritsch and J. Sambrook, Molecular Cloning: A Laboratory Manual, Cold Spring Harbor Laboratory, Cold Spring Harbor, USA, p. 383 (1982)

[20] T. Maniatis, E.F. Fritsch and J. Sambrook, Molecular Cloning: A Laboratory Manual, Cold Spring Harbor Laboratory, Cold Spring Harbor, USA, p. 387 (1982)

[21] N. Maizels, Gell 9, 431 (1976)

[22] M. Grunstein and D. Hogness, PNAS 72, 396 (1975)

[23] T. Maniatis, E.F. Fritsch and J. Sambrook, Molecular Cloning: A Laboratory Manual, Cold Spring Harbor Laboratory, Cold Spring Harbor, USA, p. 318 (1982)

[24] F. Sanger, S. Nicklen and A.R. Coulson, Proc.Natl.Acad.Sci. USA, 74 5463 (1977)

[25] J. Messing, Methods of Enzymology 101, 20 (1983)

[26] M. Poncz, D. Solowieczyk, M. Ballantine, E. Schwartz and S. Surrey, Proc.Natl.Acad.Sci. USA, 79, 4298 (1982)

[27] M.J. Zoller and M. Smith, Nucl. Acids Res. 10, 6487 (1982)

[28] T. Maniatis, E.F. Fritsch and J. Sambrook, Molecular Cloning: A Laboratory Manual, Cold Spring Harbor Laboratory, Cold Spring Harbor, USA, p. 394 (1982)

[29] T. Maniatis, E.F. Fritsch and J. Sambrook, Molecular Cloning: A Laboratory Manual, Cold Spring Harbor Laboratory, Cold Spring Harbor, USA, p. 392 (1982)

[30] Y. Shibano, A. Yamagata, N. Nakamura, T. Iizuka, H. Sugisaki and M. Takanami, Gene 34, 243 (1985)

[31] A. Krieg in: The Procaryotes, a handbook on habitats, isolation and identification of Bacteria (Eds. Starr, P.M., Stolp, H., Trueper, G.H., Balows, A. and Schlegel, H.G.), Springer Verlag New York, Heidelberg, Berlin, p. 1743 (1981)

32) H.E. Schnepf et al, The Journal of Biological Chemistry 260(10), 6273(1985)

**Patentansprüche**

**Patentansprüche für folgende Vertragsstaaten : BE, CH, LI, DE, ES, FR, GR, IT, LU, NL, SE**

1.  Ein DNA Fragment erhältlich aus *Bacillus thuringiensis* var. *kurstaki,* das durch die in Tabelle 2 wiedergegebene DNA Sequenz charakterisiert ist und eine für ein insektizid wirksames Toxinprotein kodierende DNA Sequenz umfasst, einschliesslich ausgewählter Teile besagten DNA Fragments, mit der Massgabe, dass besagte ausgewählte Teile einen kodierenden Anteil enthalten, der ausreicht, damit die insektizide Aktivität des korrespondierenden Proteinfragments erhalten bleibt und dass besagte ausgewählte Teile isoliert und nicht als Bestandteil einer chimären Genkonstruktion aus zwei oder mehreren *Bacillus thuringiensis* Genen vorliegen.

2.  Teil eines DNA Fragments gemäss Anspruch 1 mit einer Grösse von 4355 Bp, welches innerhalb des Klons pK36 vorliegt, der einkloniert in *E.coli* HB101 unter der Hinterlegungsnummer DSM 3668 hinterlegt worden ist und das durch Schneiden mit HpaI und PstI aus diesem erhältlich ist.

3.  Teil eines DNA Fragments gemäss Anspruch 1, dadurch gekennzeichnet, dass besagtes Teilfragment für das insektizid wirksame Protein MGE 1 kodiert, einschliesslich ausgewählter Teile davon, mit der Massgabe, dass die insektizide Aktivität bei den durch besagte ausgewählte Teile kodierten Toxinfragmenten erhalten bleibt und dass besagte ausgewählte Teile isoliert und nicht als Bestandteil einer chimären Genkonstruktion aus zwei oder mehreren *Bacillus thuringiensis* Genen vorliegen.

4.  Eine proteinartige Substanz, dadurch gekennzeichnet, dass sie zumindest teilweise durch ein DNA Fragment gemäss Anspruch 1 kodiert wird oder durch ausgewählte Teile davon, mit der Massgabe, dass nur dieser besagte Teil der proteinartigen Substanz aus *Bacillus thuringiensis* stammt.

5.  Eine proteinartige Substanz gemäss Anspruch 4, dadurch gekennzeichnet, dass sie zumindest teilweise durch ein DNA Fragment gemäss Anspruch 3 kodiert wird oder durch ausgewählte Teile davon.

6.  Eine proteinartige Substanz gemäss Anspruch 5, die zumindest teilweise durch die in Tabelle 3 wiedergegebene Aminosäuresequenz charakterisiert wird.

7.  Protein MGE 1, welches durch die von einem DNA-Fragment gemäss einem der Ansprüche 1 bis 3 umfasste, kodierende DNA-Sequenz kodiert wird.

8.  Protein MGE 1 gemäss Anspruch 7, charakterisiert durch die in Tabelle 3 wiedergegebene Aminosäuresequenz.

9.  Ein Fusionsprotein gemäss Anspruch 4, dadurch gekennzeichnet, dass es sich bei dem aus *Bacillus thuringiensis* stammenden Teil besagten Fusionsproteins um das Protein MGE 1 handelt.

**10.** Ein Fusionsprotein gemäss Anspruch 9, dadurch gekennzeichnet, dass das Protein, welches von dem Protein MGE 1 verschieden ist, eine herbizide Aktivität besitzt.

**11.** Ein Fusionsprotein gemäss Anspruch 9, dadurch gekennzeichnet, dass das Protein, welches von dem Protein MGE 1 verschieden ist, eine insektizide Aktivität besitzt.

**12.** Ein DNA Vektor, der ein DNA-Fragment gemäss einem der Ansprüche 1 bis 3 enthält.

**13.** Ein Vektor gemäss Anspruch 12, dadurch gekennzeichnet, dass es sich um ein Plasmid handelt.

**14.** Ein Vektor gemäss Anspruch 12, dadurch gekennzeichnet, dass es sich um einen Phagen handelt.

**15.** Ein Mikroorganismus, der ein DNA-Fragment gemäss einem der Ansprüche 1 bis 3 enthält, dadurch gekennzeichnet, dass besagter Mikroorganismus, falls er zur Gruppe des *Bacillus thuringiensis* gehört, mit einem DNA-Fragment gemäss einem der Ansprüche 1 bis 3 transformiert worden ist.

**16.** Ein Mikroorganismus gemäss Anspruch 15, dadurch gekennzeichnet, dass er zu der Spezies *Saccharomyces cerevisiae* gehört.

**17.** Ein Bioenkapsulierungs-System, das aus einem ersten Material besteht, welches vollständig in einem zweiten Material biologischer Herkunft eingeschlossen vorliegt, dadurch gekennzeichnet, dass es sich bei dem ersten Material um ein DNA-Fragment gemäss einem der Ansprüche 1 bis 3 handelt und bei dem zweiten Material um einen vollständigen Mikroorganismus, mit der Einschräukung, dass besagter Mikroorganismus, falls er zu der Gruppe des *Bacillus thuringiensis* gehört, mit einem DNA-Fragment gemäss einem der Ansprüche 1 bis 3 transformiert worden ist.

**18.** Ein Bioenkapsulierungs-System gemäss Anspruch 17, dadurch gekennzeichnet, dass es sich bei dem Mikroorganismus um *Saccharomyces cerevisiae* handelt.

**19.** Verfahren zur Herstellung eines DNA-Fragments gemäss Anspruch 1, das durch die folgenden Verfahrensschritte gekennzeichnet ist:
a) Isolierung und Lyse von *Bacillus thurigiensis* var. *kurstaki* HD1-Zellen, Abtrennen der Plasmide von dem so erhaltenen lysierten Material mit Hilfe bekannter Massnahmen und Reinigen und Dialysieren des so erhaltenen Plasmid-Materials;
b) Erstellung einer DNA-Bibliothek der *Bacillus thuringiensis* var. *kurstaki* HD1 Plasmid-DNA;
c) Klonieren der gemäss Schritt b) erhaltenen fragmentierten Plasmid DNA in einem geeigneten Vektor;
d) Screening der Klone auf die Anwesenheit von Antigen, das mit Antikörpern reagiert, die gegen das Kristallkörper-Protein von *B. thuringiensis* var. *kurstaki* hergestellt worden sind;
e) Auslesen der Klone, die spezifisch mit besagten Antikörpern reagieren; und
f) Prüfung der insektiziden Aktivität von Extrakten der gemäss Schritt e) erhaltenen Klone.

**20.** Verfahren gemäss Anspruch 19, dadurch gekennzeichnet, dass im Verfahrensschritt c) ein Plasmid als Vektor dient.

**21.** Verfahren zur Bekämpfung von Insekten, dadurch gekennzeichnet, dass man Insekten oder ihren Lebensraum mit einer insektizid wirksamen Menge einer proteinartigen Substanz gemäss einem der Ansprüche 4 bis 6 behandelt.

**22.** Verfahren zur Bekämpfung von Insekten, dadurch gekennzeichnet, dass man Insekten oder ihren Lebensraum mit einer insektizid wirksamen Menge des Proteins MGE1 gemäss einem der Ansprüche 7 bis 8 behandelt.

**23.** Verfahren zur Bekämpfung von Insekten gemäss einem der Ansprüche 21 oder 22, dadurch gekennzeichnet, dass es sich um Insekten der Ordnung *Lepidoptera* handelt.

**24.** Insektizides Mittel, dadurch gekennzeichnet, dass es eine insektizid wirksame Menge einer proteinartigen Substanz enthält, die durch ein DNA Fragment gemäss einem der Ansprüche 1 bis 3 kodiert wird.

**25.** Insektizides Mittel, dadurch gekennzeichnet, dass es eine insektizid wirksame Menge des Proteins MGE 1 gemäss einem der Ansprüche 7 bis 8 enthält.

**26.** Verfahren zur Bekämpfung von Insekten, dadurch gekennzeichnet, dass man die Insekten oder ihren Lebensraum mit einer insektizid wirksamen Menge eines Bioenkapsulierungs-Systems gemäss einem der Ansprüche 17 oder 18 behandelt.

**27.** Verfahren zur Bekämpfung von Insekten gemäss einem der Ansprüche 26 oder 27, dadurch gekennzeichnet, dass es sich um Insekten der Ordnung *Lepidoptera* handelt.

**28.** Ein Hybridvektor, enthaltend den Hefe saure-Phosphatase Promotor PHO5 und ein DNA Fragment gemäss einem der Ansprüche 1 bis 3, welches durch besagten Promoter kontrolliert wird.

**29.** Der Hefe-Stamm *Saccharomyces cerevisiae* GRF 18, von dem eine Probe unter der Hinterlegungsnummer DSM 3665 hinterlegt worden ist, dadurch gekennzeichnet, dass er mit einem Hybridvektor gemäss Anspruch 28 transformiert ist.

**30.** Der transformierte Escherichia coli-Stamm HB 101 (pK 36), von dem eine Probe unter der Hinterlegungs-Nummer DSM 3668 hinterlegt worden ist, sowie alle Abkömmlinge und Mutanten davon, die noch das DNA-Fragment gemäss einem der Ansprüche 1 bis 3 enthalten.

**31.** Verfahren zur Herstellung von Protein MGE1, dadurch gekennzeichnet, dass man die auf einem DNA Fragment gemäss Anspruch 1 befindliche kodierende Sequenz in einem geeigneten Wirtsorganismus exprimiert und das resultierende Proteinprodukt isoliert.

**32.** Verfahren gemäss Anspruch 31, dadurch gekennzeichnet, dass es sich bei besagtem Wirtsorganismus um *E. coli* oder Hefe handelt.

**33.** Verfahren gemäss Anspruch 32, dadurch gekennzeichnet, dass man besagte kodierende DNA Sequenz zunächst operabel mit einem Hefe-Promotor verknüpft

**34.** Verfahren gemäss Anspruch 33, dadurch gekennzeichnet, dass es sich bei besagtem Hefe-Promotor um den saure Phosphatase-Promotor PHO5 handelt.

**35.** Verfahren gemäss Anspruch 32, dadurch gekennzeichnet, dass es sich bei besagter Hefe um *Saccharomyces cerevisiae* handelt.

**36.** Verfahren zur Herstellung eines Mikroorganismus, der ein DNA-Fragment gemäss einem der Ansprüche 1 bis 3 enthält, dadurch gekennzeichnet, dass man besagten Mikroorganismus mit einem solchen DNA-Fragment transformiert.

**37.** Verfahren gemäss Anspruch 36, dadurch gekennzeichnet, dass es sich bei besagtem Mikroorganismus um die Hefe *Saccharomyces cerevisiae* handelt.

**38.** Verfahren zur Herstellung eines Bioenkapsulierungs-Systems, das aus einem ersten Material besteht, welches vollständig in einem zweiten Material biologischer Herkunft eingeschlossen vorliegt, wobei es sich bei dem ersten Material um ein DNA-Fragment gemäss einem der Ansprüche 1 bis 3 handelt und bei dem zweiten Material um einen vollständigen Mikroorganismus, dadurch gekennzeichnet, dass man besagtes DNA-Fragment in besagten Mikroorganismus transformiert und dort exprimiert.

**39.** Verfahren gemäss Anspruch 38, dadurch gekennzeichnet, dass es sich bei besagtem Mikroorganismus um die Hefe *Saccharomyces cerevisiae* handelt.

**40.** Verfahren zur Herstellung insektizider Mittel, dadurch gekennzeichnet, dass man ein Bioenkapsulierungssytem gemäss einem der Ansprüche 17 oder 18 enthaltend transformierte lebende oder tote Mikroorganismen oder Mischungen davon, in einer insektizid wirksamen Menge mit in der Formulie-rungstechnik üblichen Hilfsstoffen vermischt.

26

EP 0 238 441 B1

**Patentansprüche für folgenden Vertragsstaat : AT**

1. Verfahren zur Herstellung eines DNA-Fragments, das durch die in Tabelle 2 wiedergegebene DNA Sequenz charakterisiert ist und eine für ein insektizid wirksames Toxinprotein kodierende DNA Sequenz umfasst, einschliesslich ausgewählter Teile besagten DNA Fragments, mit der Massgabe, dass besagte ausgewählte Teile einen kodierenden Anteil enthalten, der ausreicht, damit die insektizide Aktivität des korrespondierenden Proteinfragments erhalten bleibt und dass besagte ausgewählte Teile isoliert und nicht als Bestandteil einer chimären Genkonstruktion aus zwei oder mehreren *Bacillus thuringiensis* Genen vorliegen, wobei besagtes Verfahren durch die folgenden Verfahrensschritte gekennzeichnet ist:

   a) Isolierung und Lyse von *Bacillus thurigiensis* var. *kurstaki* HD1-Zellen, Abtrennen der Plasmide von dem so erhaltenen lysierten Material mit Hilfe bekannter Massnahmen und Reinigen und Dialysieren des so erhaltenen Plasmid-Materials;

   b) Erstellung einer DNA-Bibliothek der *Bacillus thuringiensis* var. *kurstaki* HD1 Plasmid-DNA;

   c) Klonieren der gemäss Schritt b) erhaltenen fragmentierten Plasmid DNA in einem geeigneten Vektor;

   d) Screening der Klone auf die Anwesenheit von Antigen, das mit Antikörpern reagiert, die gegen das Kristallkörper-Protein von *B. thuringiensis* var. *kurstaki* hergestellt worden sind;

   e) Auslesen der Klone, die spezifisch mit besagten Antikörpern reagieren; und

   f) Prüfung der insektiziden Aktivität von Extrakten der gemäss Schritt e) erhaltenen Klone.

2. Verfahren gemäss Anspruch 1, dadurch gekennzeichnet, dass im Verfahrensschritt c) ein Plasmid als Vektor dient.

3. Verfahren gemäss Anspruch 1, dadurch gekennzeichnet, dass besagtes DNA Fragment eine Grösse von 4355 Bp aufweist und innerhalb des Klons pK36 vorliegt, der einkloniert in *E.coli* HB101 unter der Hinterlegungsnummer DSM 3668 hinterlegt worden ist und durch Schneiden mit HpaI und PstI aus diesem erhältlich ist.

4. Verfahren gemäss Anspruch 1, dadurch gekennzeichnet, dass besagtes DNA Fragment für das Protein MGE1 kodiert.

5. Verfahren zur Herstellung von Protein MGE1, dadurch gekennzeichnet, dass man die auf dem gemäss Anspruch 1 hergestellten DNA Fragment befindliche kodierende Sequenz in einem geeigneten Wirtsorganismus exprimiert und das resultierende Proteinprodukt isoliert.

6. Verfahren gemäss Anspruch 5, dadurch gekennzeichnet, dass es sich bei besagtem Wirtsorganismus um *E. coli* oder Hefe handelt.

7. Verfahren gemäss Anspruch 6, dadurch gekennzeichnet, dass man besagte kodierende DNA Sequenz zunächst operabel mit einem Hefe-Promotor verknüpft

8. Verfahren gemäss Anspruch 7, dadurch gekennzeichnet, dass es sich bei besagtem Hefe-Promotor um den saure Phosphatase-Promotor PHO5 handelt.

9. Verfahren gemäss Anspruch 6, dadurch gekennzeichnet, dass es sich bei besagter Hefe um *Saccharomyces cerevisiae* handelt.

10. Verfahren zur Herstellung eines Mikroorganismus, der ein gemäss einem der Ansprüche 1 bis 4 hergestelltes DNA-Fragment enthält, dadurch gekennzeichnet, dass man besagten Mikroorgansimus mit einem solchen DNA-Fragment transformiert.

11. Verfahren gemäss Anspruch 10, dadurch gekennzeichnet, dass es sich bei besagtem Mikroorganismus um die Hefe *Saccharomyces cerevisiae* handelt.

12. Verfahren zur Herstellung eines Bioenkapsulierungs-Systems, das aus einem ersten Material besteht, welches vollständig in einem zweiten Material biologischer Herkunft eingeschlossen vorliegt, wobei es sich bei dem ersten Material um ein gemäss einem der Ansprüche 1 bis 4 hergestelltes DNA-Fragment handelt und bei dem zweiten Material um einen vollständigen Mikroorganismus, dadurch gekennzeich-

27

net, dass man besagtes DNA-Fragment in besagten Mikroorganismus transformiert und dort exprimiert.

13. Verfahren gemäss Anspruch 12, dadurch gekennzeichnet, dass es sich bei besagtem Mikroorganismus um die Hefe *Saccharomyces cerevisiae* handelt.

14. Verfahren zur Bekämpfung von Insekten, dadurch gekennzeichnet, dass man Insekten oder ihren Lebensraum mit einer insektizid wirksamen Menge des gemäss einem der Ansprüche 5 bis 9 hergestellten Proteins MGE1 behandelt.

15. Verfahren zur Bekämpfung von Insekten gemäss Anspruch 14, dadurch gekennzeichnet, dass es sich um Insekten der Ordnung *Lepidoptera* handelt.

16. Verfahren zur Bekämpfung von Insekten, dadurch gekennzeichnet, dass man die Insekten oder ihren Lebensraum mit einer insektizid wirksamen Menge eines gemäss einem der Ansprüche 12 oder 13 hergestellten Bioenkapsulierungs-Systems behandelt.

17. Verfahren zur Bekämpfung von Insekten gemäss Anspruch 16, dadurch gekennzeichnet, dass es sich um Insekten der Ordnung *Lepidoptera* handelt.

18. Verfahren zur Herstellung insektizider Mittel, dadruch gekennzeichnet, dass man ein gemäss einem der Ansprüche 12 oder 13 hergestelltes Bioenkapsulierungs-System enthaltend transformierte lebende oder tote Mikroorganismen oder Mischungen davon, in einer insektizid wirksamen Menge mit den in der Formulierungstechnik üblichen Hilfsstoffen vermischt.

19. Insektizides Mittel, dadurch gekennzeichnet, dass es eine insektizid wirksame Menge einer proteinartigen Substanz enthält, die durch ein gemäss einem der Ansprüche 1 bis 4 hergestelltes DNA Fragment kodiert wird.

20. Insektizides Mittel, dadurch gekennzeichnet, dass es eine insektizid wirksame Menge des gemäss einem der Ansprüche 5 bis 9 hergestellten Proteins MGE 1 enthält.

**Claims**
**Claims for the following Contracting States : BE, CH, LI, DE, ES, FR, GR, IT, LU, NL, SE**

1. A DNA fragment obtainable from Bacillus thuringiensis var. kurstaki, which is characterized by the DNA sequence given in Table 2 and comprises a DNA sequence coding for an insecticidally active toxin protein, including selected portions of said DNA fragment, with the proviso that said selected portions contain a coding portion which is sufficient for the insecticidal activity of the corresponding protein fragment to be retained, and that said selected portions are isolated and not present as a constituent of a chimeric gene construction of two or more Bacillus thuringiensis genes.

2. Portion of a DNA fragment according to claim 1 having a size of 4355 bp, which is present within the clone pK36 which has been deposited under accession number DSM 3668 cloned into E. coli HB101 and which is obtainable from the latter by cutting with HpaI and PstI.

3. Portion of a DNA fragment according to claim 1, wherein said fragment portion codes for the insecticidally active protein MGE 1, including selected portions thereof, with the proviso that the insecticidal activity of the toxin fragments encoded by said selected portions is retained and that said selected portions are isolated and not present as a constituent of a chimeric gene construction of two or more Bacillus thuringiensis genes.

4. A proteinaceous substance which is at least partially encoded by a DNA fragment according to claim 1 or by selected portions thereof, with the proviso that only this said portion of the proteinaceous substance originates from Bacillus thuringiensis.

5. A proteinaceous substance according to claim 4, which is at least partially encoded by a DNA fragment according to claim 3 or by selected portions thereof.

6. A proteinaceous substance according to claim 5, which is at least partially characterized by the amino acid sequence given in Table 3.

7. Protein MGE 1, which is encoded by the coding DNA sequence embraced by a DNA fragment according to any one of claims 1 to 3.

8. Protein MGE 1 according to claim 7, characterized by the amino acid sequence given in Table 3.

9. A fusion protein according to claim 4, wherein the portion of said fusion protein originating from Bacillus thuringiensis is the protein MGE 1.

10. A fusion protein according to claim 9, wherein the protein which is different from the protein MGE 1 possesses herbicidal activity.

11. A fusion protein according to claim 9, wherein the protein which is different from the protein MGE 1 possesses insecticidal activity.

12. A DNA vector which contains a DNA fragment according to any one of claims 1 to 3.

13. A vector according to claim 12 which is a plasmid.

14. A vector according to claim 12 which is a phage.

15. A microorganism containing a DNA fragment according to any one of claims 1 to 3, wherein said microorganism, if it belongs to the group Bacillus thuringiensis, has been transformed with a DNA fragment according to any one of claims 1 to 3.

16. A microorganism according to claim 15 which belongs to the species Saccharomyces cerevisiae.

17. A bioencapsulation system consisting of a first material completely embedded in a second material of biological origin, wherein the first material is a DNA fragment according to any one of claims 1 to 3 and the second material is a whole microorganism, with the proviso that said microorganism, if it belongs to the group Bacillus thuringiensis, has been transformed with a DNA fragment according to any one of claims 1 to 3.

18. A bioencapsulation system according to claim 17, wherein the microorgansim is Saccharomyces cerevisiae.

19. A method for producing a DNA fragment according to claim 1, which method comprises the following steps:
a) isolating and lysing Bacillus thuringiensis var. kurstaki HD1 cells, separating the plasmids from the lysed material thus obtained by measures known per se and purifying and dialysing the plasmid material thus obtained;
b) compiling a DNA library of Bacillus thuringiensis var. kurstaki HD1 plasmid DNA;
c) cloning the fragmented plasmid DNA obtained according to step b) in a suitable vector;
d) screening the clones for the presence of antigen which reacts with antibodies which have been produced against the crystal protein of B. thuringiensis var. kurstaki;
e) selecting the clones which react specifically with said antibodies; and
f) testing the insecticidal activity of extracts of the clones obtained according to step e).

20. A method according to claim 19, wherein, in step c), the vector is a plasmid.

21. A method for combating insects which comprises applying to the insects or their habitat an insecticidally effective amount of a proteinaceous substance according to any one of claims 4 to 6.

22. A method for combating insects which comprises applying to the insects or their habitat an insecticidally effective amount of the protein MGE1 according to either of claims 7 to 8.

**23.** A method for combating insects, according to either of claims 21 or 22, wherein the insects are of the order Lepidoptera.

**24.** An insecticidal composition which comprises an insecticidally effective amount of a proteinaceous substance which is encoded by a DNA fragment according to any one of claims 1 to 3.

**25.** An insecticidal composition which comprises an insecticidally effective amount of the protein MGE 1 according to either of claims 7 to 8.

**26.** A method for combating insects which comprises applying to the insects or their habitat an insecticidally effective amount of a bioencapsulation system according to either of claims 17 or 18.

**27.** A method of combating insects according to either of claims 26 and 27, wherein the insects are of the order Lepidoptera.

**28.** A hybrid vector comprising the yeast acid phosphatase promoter PHO5 and a DNA fragment according to any one of claims 1 to 3, which is controlled by said promoter.

**29.** The yeast strain Saccharomyces cerevisiae GRF 18, a sample of which has been deposited under accession number DSM 3665, which has been transformed with a hybrid vector according to claim 28.

**30.** The transformed Escherichia coli strain HB101 (pK36), a sample of which has been deposited under accession number DSM 3668, as well as all derivatives and mutants thereof which still contain the DNA fragment according to any one of claims 1 to 3.

**31.** A method for producing protein MGE1, which comprises expressing the coding sequence located on a DNA fragment according to claim 1 in a suitable host organism and isolating the resulting protein product.

**32.** A method according to claim 31, wherein said host organism is E. coli or a yeast.

**33.** A method according to claim 32, wherein said coding DNA sequence is initially operably linked with a yeast promoter.

**34.** A method according to claim 33, wherein said yeast promoter is the acid phosphatase promoter PHO5.

**35.** A method according to claim 32, wherein said yeast is Saccharomyces cerevisiae.

**36.** A method for producing a microorganism containing a DNA fragment according to any one of claims 1 to 3, which comprises transforming said microorganism with such a DNA fragment.

**37.** A method according to claim 36, wherein said microorganism is the yeast Saccharomyces cerevisiae.

**38.** A method for producing a bioencapsulation system consisting of a first material completely embedded in a second material of biological origin, the first material being a DNA fragment according to any one of claims 1 to 3 and the second material being a whole microorganism, which comprises transforming said DNA fragment into said microorganism and expressing it there.

**39.** A method according to claim 38, wherein said microorganism is the yeast Saccharomyces cerevisiae.

**40.** A method for producing an insecticidal composition, which comprises mixing a bioencapsulation system according to either of claims 17 and 18 containing transformed living or dead microorganisms or mixtures thereof in an insecticidally effective amount with adjuvants customary in formulation technology.

EP 0 238 441 B1

**Claims for the following Contracting State : AT**

1. A method for producing a DNA fragment, which is characterized by the DNA sequence given in Table 2 and comprises a DNA sequence coding for an insecticidally active toxin protein, including selected portions of said DNA fragments, with the proviso that said selected portions contain a coding portion which is sufficient so that the insecticidal activity of the corresponding protein fragment is retained and that said selected portions are isolated and not present as constituent of a chimeric gene construction of two or more Bacillus thuringiensis genes, which method comprises the following steps:

 a) isolating and lysing Bacillus thuringiensis var. kurstaki HD1 cells, separating the plasmids from the lysed material thus obtained by measures known per se and purifying and dialysing the plasmid material thus obtained;

 b) compiling a DNA library of Bacillus thuringiensis var. kurstaki HD1 plasmid DNA;

 c) cloning the fragmented plasmid DNA obtained according to step b) in a suitable vector;

 d) screening the clones for the presence of antigen which reacts with antibodies which have been produced against the crystal protein of B. thuringiensis var. kurstaki;

 e) selecting the clones which react specifically with said antibodies; and

 f) testing the insecticidal activity of extracts of the clones obtained according to step e).

2. A method according to claim 1, wherein, in step c), the vector is a plasmid.

3. A method according to claim 1, wherein said DNA fragment has a size of 4355 bp and is present within the clone pK36 which has been deposited under accession number DSM 3668 cloned into E. coli HB101 and which is obtainable from the latter by cutting with HpaI and PstI.

4. A method according to claim 1 wherein said DNA fragment codes for the protein MGE1.

5. A method for producing protein MGE1, which comprises expressing the coding sequence on the DNA fragment produced according to claim 1 in a suitable host organism and isolating the resulting protein product.

6. A method according to claim 5, wherein said host organism is E. coli or a yeast.

7. A method according to claim 6, wherein said coding DNA sequence is initially operably linked with a yeast promoter.

8. A method according to claim 7, wherein said yeast promoter is the acid phosphatase promoter PHO5.

9. A method according to claim 6, wherein said yeast is Saccharomyces cerevisiae.

10. A method for producing a microorganism containing a DNA fragment produced according to any one of claims 1 to 4 which comprises transforming said microorganism with such a DNA fragment.

11. A method according to claim 10, wherein said microorganism is the yeast Saccharomyces cerevisiae.

12. A method for producing a bioencapsulation system consisting of a first material completely embedded in a second material of biological origin, the first material being a DNA fragment produced according to any one of claims 1 to 4 and the second material being a whole microorganism, which comprises transforming said DNA fragment into said microorganism and expressing it there.

13. A method according to claim 12, wherein said microorganism is the yeast Saccharomyces cerevisiae.

14. A method for combating insects which comprises applying to the insects or their habitat an insecticidally effective amount of the protein MGE1 produced according to any one of claims 5 to 9.

15. A method for combating insects according to claim 14, wherein the insects are of the order Lepidoptera.

31

16. A method for combating insects which comprises applying to the insects or their habitat an insecticidally effective amount of a bioencapsulation system produced according to either of claims 12 or 13.

17. A method of combating insects according to claim 16, wherein the insects are of the order Lepidoptera.

18. A method for producing an insecticidal composition, which comprises mixing a bioencapsulation system produced according to either of claims 12 or 13 containing transformed living or dead microorganisms or mixtures thereof in an insecticidally effective amount with adjuvants customary in formulation technology.

19. An insecticidal composition which comprises an insecticidally effective amount of a proteinaceous substance which is encoded by a DNA fragment produced according to any one of claims 1 to 4.

20. An insecticidal composition which comprises an insecticidally effective amount of the protein MGE 1 produced according to any one of claims 5 to 9.

**Revendications**
**Revendications pour les Etats contractants suivants : BE, CH, LI, DE, ES, FR, GR, IT, LU, NL, SE**

1. Un fragment d'ADN obtenu à partir de Bacillus thuringiensis var. kurstaki, caractérisé par la séquence d'ADN figurant dans le Tableau 2 et comprenant une séquence d'ADN codant pour la toxine protéique ayant une activité insecticide, y compris les portions tronquées dudit fragment d'ADN, sous réserve que lesdites portions tronquées contiennent une partie codante suffisante pour conserver l'activité insecticide du fragment protéinique correspondant et que lesdites portions tronquées se présentent isolées et non comme constituant d'une construction de gènes chimère constituée de deux ou plusieurs gènes de Bacillus thuringiensis.

2. La portion d'un fragment d'ADN selon la revendication 1 ayant une taille de 4355 pb, se trouvant à l'intérieur du clone pK36 qui cloné dans E. coli HB101 a été déposé sous le numéro DSM 3668 et étant obtenu à partir de celui-ci par clivage avec HpaI et PstI.

3. La portion d'un fragment d'ADN selon la revendication 1, caractérisée en ce que ladite portion de fragment code pour la protéine MGE 1 ayant une activité insecticide, y compris ses portions tronquées, sous réserve que l'activité insecticide soit Conservée pour les fragments de toxine codée par lesdites portions tronquées et que lesdites portions tronquées se présentent isolées et non comme constituant d'une construction de gènes chimère constituée de deux ou plusieurs gènes de Bacillus thuringiensis.

4. Une substance protéinique caractérisée en ce qu'elle est codée au moins partiellement par un fragment d'ADN selon la revendication 1 ou par ses portions tronquées, sous réserve que seulement ladite portion de la substance protéinique provienne de Bacillus thuringiensis.

5. Une substance protéinique selon la revendication 4, caractérisée en ce qu'elle est codée au moins partiellement par un fragment d'ADN selon la revendication 3 ou par ses parties tronquées.

6. Une substance protéinique selon la revendication 5, caractérisée au moins partiellement par la séquence d'amino-acides donnée dans le Tableau 3.

7. La protéine MGE 1 codée par la séquence d'ADN codante comprise dans un fragment d'ADN selon l'une des revendications 1 à 3.

8. La protéine MGE 1 selon la revendication 7, caractérisée par la séquence d'amino-acides donnée dans le Tableau 3.

9. Une protéine de fusion selon la revendication 4, caractérisée en ce que la portion provenant du Bacillus thuringiensis de ladite protéine de fusion est la protéine MGE 1.

10. Une protéine de fusion selon la revendication 9, caractérisée en ce que la protéine qui est différente de la protéine MGE 1, présente une activité herbicide.

**11.** Une protéine de fusion selon la revendication 9, caractérisée en ce que la protéine qui est différente de la protéine MGE 1, présente une activité insecticide.

**12.** Un vecteur d'ADN contenant un fragment d'ADN selon l'une des revendications 1 à 3.

**13.** Un vecteur selon la revendication 12, caractérisé en ce que ledit vecteur est un plasmide.

**14.** Un vecteur selon la revendication 12, caractérisé en ce que ledit vecteur est un phage.

**15.** Un microorganisme contenant un fragment d'ADN selon l'une des revendications 1 à 3, caractérisé en ce que ledit microorganisme, dans le cas où il appartient au groupe Bacillus thuringiensis, est transformé avec un fragment d'ADN selon l'une des revendications 1 à 3.

**16.** Un microorganisme selon la revendication 15, caractérisé en ce qu'il appartient à l'espèce Saccharomyces cerevisiae.

**17.** Un système de bioencapsulation constitué d'un premier matériau se trouvant totalement incorporé dans un second matériau d'origine biologique, caractérisé en ce que le premier matériau est un fragment d'ADN selon l'une des revendications 1 à 3 et en ce que le second matériau est un microorganisme entier, sous réserve que ledit microorganisme, dans le cas où il appartient au groupe du Bacillus thuringiensis, ait été transformé avec le fragment d'ADN' selon l'une des revendications 1 à 3.

**18.** Un système de bioencapsulation selon la revendication 17, caractérisé en ce que le microorganisme est Saccharomyces cerevisiae.

**19.** Procédé pour la préparation d'un fragment d'ADN selon la revendication 1, caractérisé par les étapes suivantes :

a) isolement et lyse de cellules de Bacillus thuringiensis var. kurstaki HD1, séparation des plasmides du matériau lysé ainsi obtenu par des mesures connues, purification et dialyse du matériau plasmidique ainsi obtenu;

b) préparation d'une bibliothèque d'ADN de l'ADN plasmidique de Bacillus thuringiensis var. kurstaki HD1;

c) clonage de l'ADN plasmidique fragmenté obtenu selon l'étape b) dans un vecteur approprié;

d) sélection des clones pour la présence d'antigènes réagissant avec des anticorps produits contre la protéine cristalline de B. thuringiensis var. kurstaki;

e) sélection des clones réagissant spécifiquement avec lesdits anticorps et

f) contrôle de l'activité insecticide des extraits des clones obtenus selon l'étape e).

**20.** Procédé selon la revendication 19, caractérisé en ce qu'à l'étape c) du procédé un plasmide sert de vecteur.

**21.** Procédé pour lutter contre les insectes, caractérisé en ce que l'on traite les insectes ou leurs habitats avec une quantité insecticide efficace d'une substance protéinique selon l'une des revendications 4 à 6.

**22.** Procédé pour lutter contre les insectes, caractérisé en ce que l'on traite les insectes ou leurs habitats avec une quantité insecticide efficace de la protéine MGE 1 selon l'une des revendications 7 et 8.

**23.** Procédé pour lutter contre les insectes selon l'une des revendications 21 et 22, caractérisé en ce que lesdits insectes sont des insectes de l'ordre des lépidoptères.

**24.** Composition insecticide, caractérisée en ce qu'elle contient une quantité insecticide efficace d'une substance protéinique, codée par un fragment d'ADN selon l'une des revendications 1 à 3.

**25.** Composition insecticide caractérisée en ce qu'elle contient une quantité insecticide efficace de la protéine MGE 1 selon l'une des revendications 7 et 8.

**26.** Procédé pour lutter contre les insectes, caractérisé en ce que l'on traite les insectes ou leurs habitats avec une quantité insecticide efficace d'un système de bioencapsulation selon l'une des revendications 17 et 18.

**27.** Procédé pour lutter contre les insectes selon l'une des revendications 26 et 27, caractérisé en ce que lesdits insectes sont des insectes de l'ordre des lépidoptères.

**28.** Un vecteur hybride contenant le promoteur phosphatase acide de la levure PHO5 et un fragment d'ADN selon l'une des revendications 1 à 3 contrôlé par ledit promoteur.

**29.** La souche de levure Saccharomyces cerevisiae GRF18 dont un échantillon a été déposé sous le numéro DSM 3665, caractérisée en ce qu'elle est transformée avec un vecteur hybride selon la revendication 28.

**30.** La souche d'Escherichia coli HB101 (pK36) transformée dont un échantillon a été déposé sous le numéro DSM 3668 ainsi que tous ses dérivés et mutants contenant encore le fragment d'ADN selon l'une des revendications 1 à 3.

**31.** Procédé pour la préparation de la protéine MGE 1, caractérisé en ce que l'on exprime la séquence codante se trouvant sur un fragment d'ADN selon la revendication 1 dans un organisme hôte approprié et en ce que l'on isole le produit protéinique résultant.

**32.** Procédé selon la revendication 31, caractérisé en ce que ledit organisme hôte est E. coli ou une levure.

**33.** Procédé selon la revendication 32, caractérisé en ce que ladite séquence d'ADN codante est tout d'abord liée, de façon opérationnelle, à un promoteur de la levure.

**34.** Procédé selon la revendication 33, caractérisé en ce que ledit promoteur de la levure est le promoteur phosphatase acide PHO5.

**35.** Procédé selon la revendication 32, caractérisé en ce que ladite levure est Saccharomyces cerevisiae.

**36.** Procédé pour la préparation d'un microorganisme contenant un fragment d'ADN selon l'une des revendications 1 à 3, caractérisé en ce que l'on transforme ledit microorganisme avec un tel fragment d'ADN.

**37.** Procédé selon la revendication 36, caractérisé en ce que ledit microorganisme est la levure Saccharomyces cerevisiae.

**38.** Procédé pour la préparation d'un système de bioencapsulation constitué d'un premier matériau, se présentant totalement incorporé dans un second matériau d'origine biologique, le premier matériau étant un fragment d'ADN selon l'une des revendications 1 à 3 et le second matériau étant un microorganisme entier, caractérisé en ce que l'on transforme ledit fragment d'ADN dans ledit microorganisme et en ce qu'on l'exprime dans celui-ci.

**39.** Procédé selon la revendication 38, caractérisé en ce que ledit microorganisme est la levure Saccharomyces cerevisiae.

**40.** Procédé pour la préparation d'une composition insecticide, caractérisé en ce que l'on mélange un système de bioencapsulation selon l'une des revendications 17 et 18 contenant des microorganismes vivants ou morts transformés ou des mélanges de ceux-ci, en une quantité insecticide efficace avec des adjuvants usuels dans la technique de formulation.

**Revendications pour l'Etat contractant suivant : AT**

**1.** Procédé pour la préparation d'un fragment d'ADN, caractérisé par la séquence d'ADN figurant dans le Tableau 2 et comprenant une séquence d'ADN codant pour la toxine protéique ayant une activité insecticide, y compris les portions tronquées dudit fragment d'ADN, sous réserve que lesdites portions tronquées contiennent une partie codante suffisante pour conserver l'activité insecticide du fragment protéinique correspondant et que lesdites portions tronquées se présentent isolées et non comme constituant d'une construction de gènes chimère constituée de deux ou plusieurs gènes de Bacillus thuringiensis, ledit procédé étant caractérisé par les étapes suivantes :

    a) isolement et lyse de cellules de Bacillus thuringiensis var. kurstaki HD1, séparation des plasmides du matériau lysé ainsi obtenu par des mesures connues, purification et dialyse du matériau plasmidique ainsi obtenu;

    b) préparation d'une bibliothèque d'ADN de l'ADN plasmidique de Bacillus thuringiensis var. kurstaki HD1;

    c) clonage de l'ADN plasmidique fragmenté obtenu selon l'étape b) du procédé dans un vecteur approprié;

    d) sélection des clones pour la présence d'antigènes réagissant avec des anticorps produits contre la protéine cristalline de B. thuringiensis var. kurstaki;

    e) sélection des clones réagissant spécifiquement avec lesdits anticorps et

    f) contrôle de l'activité insecticide des extraits des clones obtenus selon l'étape e).

2. Procédé selon la revendication 1, caractérisé en ce qu'à l'étape c) du procédé un plasmide sert de vecteur.

3. Procédé selon la revendication 1, caractérisé en ce que ledit fragment d'ADN a une taille de 4355 pb et se trouve à l'intérieur du clone pK36 qui cloné dans E. coli HB101 a été déposé sous le numéro DSM 3668 et est obtenu à partir de celui-ci par clivage avec HpaI et PstI.

4. Procédé selon la revendication 1, caractérisé en ce que ledit fragment d'ADN code pour la protéine MGE 1.

5. Procédé pour la préparation de la protéine MGE 1, caractérisé en ce que l'on exprime la séquence codante se trouvant sur le fragment d'ADN produit selon la revendication 1 dans un organisme hôte approprié et en ce que l'on isole le produit protéinique résultant.

6. Procédé selon la revendication 5, caractérisé en ce que ledit organisme hôte est E. coli ou une levure.

7. Procédé selon la revendication 6, caractérisé en ce que l'on lie tout d'abord ladite séquence d'ADN codante, de façon opérationnelle, à un promoteur de levure.

8. Procédé selon la revendication 7, caractérisé en ce que ledit promoteur de levure est le promoteur phosphatase acide PHO5.

9. Procédé selon la revendication 6, caractérisé en ce que ladite levure est Saccharomyces cerevisiae.

10. Procédé pour la préparation d'un microorganisme contenant un fragment d'ADN produit selon l'une des revendications 1 à 4, caractérisé en ce que l'on transforme ledit microorganisme avec un tel fragment d'ADN.

11. Procédé selon la revendication 10, caractérisé en ce que ledit microorganisme est la levure Saccharomyces cerevisiae.

12. Procédé pour la préparation d'un système de bioencapsulation constitué d'un premier matériau, se présentant totalement incorporé dans un second matériau d'origine biologique, le premier matériau étant un fragment d'ADN selon l'une des revendications 1 à 4 et le second matériau étant un microorganisme entier, caractérisé en ce que l'on transforme ledit fragment d'ADN dans ledit microorganisme et en ce qu'on l'exprime dans celui-ci.

13. Procédé selon la revendication 12, caractérisé en ce que ledit microorganisme est la levure Saccharomyces cerevisiae.

14. Procédé pour lutter contre les insectes, caractérisé en ce que l'on traite les insectes ou leurs habitats avec une quantité insecticide efficace de la protéine MGE 1 préparée selon l'une des revendications 5 à 9.

**15.** Procédé pour lutter contre les insectes selon la revendication 14, caractérisé en ce que lesdits insectes sont de l'ordre des lépidoptères.

**16.** Procédé pour lutter contre les insectes, caractérisé en ce que l'on traite les insectes ou leurs habitats avec une quantité insecticide efficace d'un système de bioencapsulation préparé selon l'une des revendications 12 et 13.

**17.** Procédé pour lutter contre les insectes selon la revendication 16, caractérisé en ce que lesdits insectes sont de l'ordre des lépidoptères.

**18.** Procédé pour la préparation d'une composition insecticide, caractérisé en ce que l'on mélange un système de bioencapsulation préparé selon l'une des revendications 12 et 13 contenant des microorganismes morts ou vivants transformés ou des mélanges de ceux-ci en une quantité insecticide efficace avec les adjuvants usuels dans la technique de formulation.

**19.** Composition insecticide, caractérisée en ce qu'elle contient une quantité insecticide efficace d'une substance protéinique codée par un fragment d'ADN produit selon l'une des revendications 1 à 4.

**20.** Composition insecticide caractérisée en ce qu'elle contient une quantité insecticide efficace de la protéine MGE 1 préparée selon l'une des revendications 5 à 9.

1) Verdauung mit
   Endonuklease B
2) Bal-31

Verdauung mit
Endonuklease A

Verknüpfung, Transformation und Herstellung.
einzelsträngiger DNA
(Matritze)

Abb. 1 modifiziert nach M. Poncz et al. [26]. Konstruktion der
Deletions-Mutanten-Bibliothek. Vorgehensweise: 1, das Insert (dicke
Linie) wird in die A-Stelle des M13, die kohesive Enden aufweist,
eingespleisst; 2, nach Linearisierung an der B-Stelle wird die
Phagen DNA mit Bal-31 unterschiedlich lange verdaut [die gestrichelte Linie gibt das Ausmass der Bal-31-Verdauung in bezug auf das
Insert (dicke Linie) und auf M13 (dünne Linie) an]; 3, das Verdauungsprodukt wird an der A-Stelle gespalten und das durch Bal-31
induzierte Kontinuum des Inserts isoliert. Es entsteht auf diese
Weise eine ganze Familie von Fragmenten unterschiedlicher Grösse,
die jeweils ein Bal-31 induziertes glattes Ende und ein kohesives
Ende an der A-Stelle aufweisen; 4, die Fragmente werden so in M13
subkloniert, dass das glatte Ende proximal der Primer-Stelle P zu
liegen kommt, die für die DNA-Sequenzanalyse verwendet wird.
X und C = glatte Enden.

Tabelle 1

|  | A | B | C | M13mpi |
|---|---|---|---|---|
| (BamHI) HpaI-HindIII | | | | |
| ←————————a) | BamHI | HindIII | HincII | 8 |
| ————————→ | HindIII | BamHI | SmaI | 9 |
| EcoRI-PstI | | | | |
| ←———————— | EcoRI | PstI | SmaI | 8 |
| ————————→ | PstI | EcoRI | SmaI | 9 |

a) gemäss dem angegebenen Beispiel

Tabelle 2: Nukleotid-Sequenz der für das Protein MGE1 kodierenden DNA

```
         10         20         30         40         50         60
GTTAACACCC TGGGTCAAAA ATTGATATTT AGTAAAATTA GTTGCACTTT GTGCATTTTT

         70         80         90        100        110        120
TCATAAGATG AGTCATATGT TTTAAATTGT AGTAATGAAA AACAGTATTA TATCATAATG

        130        140        150        160        170        180
AATTGGTATC TTAATAAAAG AGATGGAGGT AACTTATGGA TAACAATCCG AACATCAATG

        190        200        210        220        230        240
AATGCATTCC TTATAATTGT TTAAGTAACC CTGAAGTAGA AGTATTAGGT GGAGAAAGAA

        250        260        270        280        290        300
TAGAAACTGG TTACACCCCA ATCGATATTT CCTTGTCGCT AACGCAATTT CTTTTGAGTG

        310        320        330        340        350        360
AATTTGTTCC CGGTGCTGGA TTTGTGTTAG GACTAGTTGA TATAATATGG GGAATTTTTG

        370        380        390        400        410        420
GTCCCTCTCA ATGGGACGCA TTTCTTGTAC AAATTGAACA GTTAATTAAC CAAAGAATAG

        430        440        450        460        470        480
AAGAATTCGC TAGGAACCAA GCCATTTCTA GATTAGAAGG ACTAAGCAAT CTTTATCAAA

        490        500        510        520        530        540
TTTACGCAGA ATCTTTTAGA GAGTGGGAAG CAGATCCTAC TAATCCAGCA TTAAGAGAAG

        550        560        570        580        590        600
AGATGCGTAT TCAATTCAAT GACATGAACA GTGCCCTTAC AACCGCTATT CCTCTTTTTG
```

Tabelle 2: (Fortsetzung)

```
         610        620        630        640        650        660
  CAGTTCAAAA TTATCAAGTT CCTCTTTTAT CAGTATATGT TCAAGCTGCA AATTACATT

         670        680        690        700        710        720
  TATCAGTTTT GAGAGATGTT TCAGTGTTTG GACAAAGGTG GGGATTTGAT GCCGGGACTA

         730        740        750        760        770        780
  TCAATAGTCG TTATAATGAT TTAACTAGGC TTATTGGCAA CTATACAGAT CATGCTGTAC

         790        800        810        820        830        840
  GCTGGTACAA TACGGGATTA GAGCGTGTAT GGGGACCGGA TTCTAGAGAT TGGATAAGAT

         850        860        870        880        890        900
  ATAATCAATT TAGAAGAGAA TTAACACTAA CTGTATTAGA TATCGTTTCT CTATTCCGA

         910        920        930        940        950        960
  ACTATGATAG TAGAACGTAT CCAATTCGAA CAGTTTCCCA ATTAACAAGA GAAATTTATA

         970        980        990       1000       1010       1020
  CAAACCCAGT ATTAGAAAAT TTTGATGGTA GTTTTCGAGG CTCGGCTCAG GGCATAGAAG

        1030       1040       1050       1060       1070       1080
  GAAGTATTAG GAGTCCACAT TTGATGGATA TACTTAACAG TATAACCATC TATACGGATG

        1090       1100       1110       1120       1130       1140
  CTCATAGAGG AGAATATTAT TGGTCAGGGC ATCAAATAAT GGCTTCTCCT GTAGGGTTTT

        1150       1160       1170       1180       1190       1200
  CGGGGCCAGA ATTCACTTTT CCGCTATATG GAACTATGGG AAATGCAGCT CCACAACAAC
```

Tabelle 2: (Fortsetzung)

```
      1210        1220        1230        1240        1250        1260
GTATTGTTGC  TCAACTAGGT  CAGGGCGTGT  ATAGAACATT  ATCGTCCACT  TTATATAGAA

      1270        1280        1290        1300        1310        1320
GACCTTTTAA  TATAGGGATA  AATAATCAAC  AACTATCTGT  TCTTGACGGG  ACAGAATTTG

      1330        1340        1350        1360        1370        1380
CTTATGGAAC  CTCCTCAAAT  TTGCCATCCG  CTGTATACAG  AAAAAGCGGA  ACGGTAGATT

      1390        1400        1410        1420        1430        1440
CGCTGGATGA  AATACCGCCA  CAGAATAACA  ACGTGCCACC  TAGGCAAGGA  TTTAGTCATC

      1450        1460        1470        1480        1490        1500
GATTAAGCCA  TGTTTCAATG  TTTCGTTCAG  GCTTTAGTAA  TAGTAGTGTA  AGTATAATAA

      1510        1520        1530        1540        1550        1560
GAGCTCCTAT  GTTCTCTTGG  ATACATCGTA  GTGCTGAATT  TAATAATATA  ATTCCTTCAT

      1570        1580        1590        1600        1610        1620
CACAAATTAC  ACAAATACCT  TTAACAAAAT  CTACTAATCT  TGGCTCTGGA  ACTTCTGTCG

      1630        1640        1650        1660        1670        1680
TTAAAGGACC  AGGATTTACA  GGAGGAGATA  TTCTTCGAAG  AACTTCACCT  GGCCAGATTT

      1690        1700        1710        1720        1730        1740
CAACCTTAAG  AGTAAATATT  ACTGCACCAT  TATCACAAAG  ATATCGGGTA  AGAATTCGCT

      1750        1760        1770        1780        1790        1800
ACGCTTCTAC.CACAAATTTA  CAATTCCATA  CATCAATTGA  CGGAAGACCT  ATTAATCAGG
```

EP 0 238 441 B1

<u>Tabelle 2</u>: (Fortsetzung)

```
    1010       1020       1030       1040       1050       1060
GGAATTTTTC AGCAACTATG AGTAGTGGGA GTAATTTACA GTCCGGAAGC TTTAGGACTG

    1070       1080       1090       1100       1110       1120
TAGGTTTTAC TACTCCGTTT AACTTTTCAA ATGGATCAAG TGTATTTACG TTAAGTGCTC

    1130       1140       1150       1160       1170       1180
ATGTCTTCAA TTCAGGCAAT GAAGTTTATA TAGATCGAAT TGAATTTGTT CCGGCAGAAG

    1190       1200       1210       1220       1230       1240
TAACCTTTGA GGCAGAATAT GATTTAGAAA GAGCACAAAA GGCGGTGAAT GAGCTGTTTA

    1250       1260       1270       1280       1290       1300
CTTCTTCCAA TCAAATCGGG TTAAAAACAG ATGTGACGGA TTATCATATT GATCAAGTAT

    1310       1320       1330       1340       1350       1360
CCAATTTAGT TGAGTGTTTA TCTGATGAAT TTTGTCTGGA TGAAAAAAAA GAATTGTCCG

    1370       1380       1390       1400       1410       1420
AGAAAGTCAA ACATGCGAAG CGACTTAGTG ATGAGCGGAA TTTACTTCAA GATCCAAACT

    1230       1240       1250       1260       1270       1280
TTAGAGGGAT CAATAGACAA CTAGACCGTG GCTGGAGAGG AAGTACGGAT ATTACCATCC

    1290       1300       1310       1320       1330       1340
AAGGAGGCGA TGACGTATTC AAAGAGAATT ACGTTACGCT ATTGGGTACC TTTGATGAGT

    1350       1360       1370       1380       1390       1400
GCTATCCAAC GTATTTATAT CAAAAAATAG ATGAGTCGAA ATTAAAAGCC TATACCCGTT
```

EP 0 238 441 B1

Tabelle 2: (Fortsetzung)

```
        2410        2420        2430        2440        2450        2460
ACCAATTAAG AGGGTATATC GAAGATAGTC AAGACTTAGA AATCTATTTA ATTCGCTACA

        2470        2480        2490        2500        2510        2520
ATGCCAAACA CGAAACAGTA AATGTGCCAG GTACGGGTTC CTTATGGCCG CTTTCAGCCC

        2530        2540        2550        2560        2570        2580
CAAGTCCAAT CGGAAAATGT GCCCATCATT CCCATCATTT CTCCTTGGAC ATTGATGTTG

        2590        2600        2610        2620        2630        2640
GATGTACAGA CTTAAATGAG GACTTAGGTG TATGGGTGAT ATTCAAGATT AAGACGCAAG

        2650        2660        2670        2680        2690        2700
ATGGCCATGC AAGACTAGGA AATCTAGAAT TTCTCGAAGA GAAACCATTA GTAGGAGAAG

        2710        2720        2730        2740        2750        2760
CACTAGCTCG TGTGAAAAGA GCGGAGAAAA AATGGAGAGA CAAACGTGAA AAATTGGAAT

        2770        2780        2790        2800        2810        2820
GGGAAACAAA TATTGTTTAT AAAGAGGCAA AAGAATCTGT AGATGCTTTA TTTGTAAACT

        2830        2840        2850        2860        2870        2880
CTCAATATGA TAGATTACAA GCGGATACCA ACATCGCGAT GATTCATGCG GCAGATAAAC

        2890        2900        2910        2920        2930        2940
GCGTTCATAG CATTCGAGAA GCTTATCTGC CTGAGCTGTC TGTGATTCCG GGTGTCAATG

        2950        2960        2970        2980        2990        3000
CGGCTATTTT TGAAGAATTA GAAGGGCGTA TTTTCACTGC ATTCTCCCTA TATGATGCGA
```

EP 0 238 441 B1

Tabelle 2: (Fortsetzung)

```
       3010        3020        3030        3040        3050        3060
  GAAATGTCAT TAAAAATGGT GATTTTAATA ATGGCTTATC CTGCTGGAAC GTGAAAGGGC

       3070        3080        3090        3100        3110        3120
  ATGTAGATGT AGAAGAACAA AACAACCACC GTTCGGTCCT TGTTGTTCCG GAATGGGAAG

       3130        3140        3150        3160        3170        3180
  CAGAAGTGTC ACAAGAAGTT CGTGTCTGTC CGGGTCGTGG CTATATCCTT CGTGTCACAG

       3190        3200        3210        3220        3230        3240
  CGTACAAGGA GGGATATGGA GAAGGTTGCG TAACCATTCA TGAGATCGAG AACAATACAG

       3250        3260        3270        3280        3290        3300
  ACGAACTGAA GTTTAGCAAC TGTGTAGAAG AGGAAGTATA TCCAAACAAC ACGGTAACGT

       3310        3320        3330        3340        3350        3360
  GTAATGATTA TACTGCGACT CAAGAAGAAT ATGAGGGTAC GTACACTTCT CGTAATCGAG

       3370        3380        3390        3400        3410        3420
  GATATGACGG AGCCTATGAA AGCAATTCTT CTGTACCAGC TGATTATGCA TCAGCCTATG

       3430        3440        3450        3460        3470        3480
  AAGAAAAAGC ATATACAGAT GGACGAAGAG ACAATCCTTG TGAATCTAAC AGAGGATATG

       3490        3500        3510        3520        3530        3540
  GGGATTACAC ACCACTACCA GCTGGCTATG TGACAAAAGA ATTAGAGTAC TTCCCAGAAA

       3550        3560        3570        3580        3590        3600
  CCGATAAGGT ATGGATTGAG ATCGGAGAAA CGGAAGGAAC ATTCATCGTG GACAGCGTGG
```

EP 0 238 441 B1

Tabelle 2: (Fortsetzung)

```
   3610       3620       3630       3640       3650       3660
AATTACTTCT TATGGAGGAA TAATATATGC TTTAAAATGT AAGGTGTGCA AATAAAGAAT

   3670       3600       3690       3700       3710       3720
GATTACTGAC TTGTATTGAC AGATAAATAA GGAAATTTTT ATATGAATAA AAAACGGGCA

   3730       3740       3750       3760       3770       3780
TCACTCTTAA AAGAATGATG TCCGTTTTTT GTATGATTTA ACGAGTGATA TTTAAATGTT

   3790       3800       3810       3820       3830       3840
TTTTTTGCGA AGGCTTTACT TAACGGGGTA CCGCCACATG CCCATCAACT TAAGAATTTG

   3850       3860       3870       3880       3890       3900
CACTACCCCC AAGTGTCAAA AAACGTTATT CTTTCTAAAA AGCTAGCTAG AAAGGATGAC

   3910       3920       3930       3940       3950       3960
ATTTTTTATG AATCTTTCAA TTCAAGATGA ATTACAACTA TTTTCTGAAG AGCTGTATCG

   3970       3980       3990       4000       4010       4020
TCATTTAACC CCTTCTCTTT TGGAAGAACT CGCTAAAGAA TTAGGTTTTG TAAAAAGAAA

   4030       4040       4050       4060       4070       4080
ACGAAAGTTT TCAGGAAATG AATTAGCTAC CATATGTATC TGGGGCAGTC AACGTACAGC

   4090       4100       4110       4120       4130       4140
GAGTGATTCT CTCGTTCGAC TATGCAGTCA ATTACACGCC GCCACAGCAC TCTTATGAGT

   4150       4160       4170       4180       4190       4200
CCAGAAGGAC TCAATAAACG CTTTGATAAA AAAGCGGTTG AATTTTTGAA ATATATTTTT
```

EP 0 238 441 B1

Tabelle 2: (Fortsetzung)

```
     4210        4220         4230        4240        4250        4260
TCTGCATTAT GGAAAAGTAA ACTTTGTAAA ACATCAGCCA TTTCAAGTGC AGCACTCACG

     4270        4280         4290        4300        4310        4320
TATTTTCAAC GAATCCGTAT TTTAGATGCG ACGATTTTCC AAGTACCGAA ACATTTAGCA

     4330        4340         4350        4360
CATGTATATC CTGGGTCAGG TGGTTGTGCA CAAACTGCAG
```

Tabelle 3: Aminosäuresequenz des Proteins MGE1

LIMITS:   156   3623

```
                                               185                                          215
ATG GAT AAC AAT CCG AAC ATC AAT GAA TGC ATT CCT TAT AAT TGT TTA AGT AAC CCT GAA
Met Asp Asn Asn Pro Asn Ile Asn Glu Cys Ile Pro Tyr Asn Cys Leu Ser Asn Pro Glu

                                               245                                          275
GTA GAA GTA TTA GGT GGA GAA AGA ATA GAA ACT GGT TAC ACC CCA ATC GAT ATT TCC TTG
Val Glu Val Leu Gly Gly Glu Arg Ile Glu Thr Gly Tyr Thr Pro Ile Asp Ile Ser Leu

                                               305                                          335
TCG CTA ACG CAA TTT CTT TTG AGT GAA TTT GTT CCC GGT GCT GGA TTT GTG TTA GGA CTA
Ser Leu Thr Gln Phe Leu Leu Ser Glu Phe Val Pro Gly Ala Gly Phe Val Leu Gly Leu

                                               365                                          395
GTT GAT ATA ATA TGG GGA ATT TTT GGT CCC TCT CAA TGG GAC GCA TTT CTT GTA CAA ATT
Val Asp Ile Ile Trp Gly Ile Phe Gly Pro Ser Gln Trp Asp Ala Phe Leu Val Gln Ile

                                               425                                          455
GAA CAG TTA ATT AAC CAA AGA ATA GAA GAA TTC GCT AGG AAC CAA GCC ATT TCT AGA TTA
Glu Gln Leu Ile Asn Gln Arg Ile Glu Glu Phe Ala Arg Asn Gln Ala Ile Ser Arg Leu

                                               485                                          515
GAA GGA CTA AGC AAT CTT TAT CAA ATT TAC GCA GAA TCT TTT AGA GAG TGG GAA GCA GAT
Glu Gly Leu Ser Asn Leu Tyr Gln Ile Tyr Ala Glu Ser Phe Arg Glu Trp Glu Ala Asp

                                               545                                          575
CCT ACT AAT CCA GCA TTA AGA GAA GAG ATG CGT ATT CAA TTC AAT GAC ATG AAC AGT GCC
Pro Thr Asn Pro Ala Leu Arg Glu Glu Met Arg Ile Gln Phe Asn Asp Met Asn Ser Ala
```

EP 0 238 441 B1

```
                                                605                                               635
CTT ACA ACC GCT ATT CCT CTT TTT GCA GTT CAA AAT TAT CAA GTT CCT CTT TTA TCA GTA
Leu Thr Thr Ala Ile Pro Leu Phe Ala Val Gln Asn Tyr Gln Val Pro Leu Leu Ser Val

                                                665                                               695
TAT GTT CAA GCT GCA AAT TTA CAT TTA TCA GTT TTG AGA GAT GTT TCA GTG TTT GGA CAA
Tyr Val Gln Ala Ala Asn Leu His Leu Ser Val Leu Arg Asp Val Ser Val Phe Gly Gln

                                                725                                               755
AGG TGG GGA TTT GAT GCC GCG ACT ATC AAT AGT CGT TAT AAT GAT TTA ACT AGG CTT ATT
Arg Trp Gly Phe Asp Ala Ala Thr Ile Asn Ser Arg Tyr Asn Asp Leu Thr Arg Leu Ile

                                                785                                               815
GGC AAC TAT ACA GAT CAT GCT GTA CGC TGG TAC AAT ACG GGA TTA GAG CGT GTA TGG GGA
Gly Asn Tyr Thr Asp His Ala Val Arg Trp Tyr Asn Thr Gly Leu Glu Arg Val Trp Gly

                                                845                                               875
CCG GAT TCT AGA GAT TGG ATA AGA TAT AAT CAA TTT AGA AGA GAA TTA ACA CTA ACT GTA
Pro Asp Ser Arg Asp Trp Ile Arg Tyr Asn Gln Phe Arg Arg Glu Leu Thr Leu Thr Val

                                                905                                               935
TTA GAT ATC GTT TCT CTA TTT CCG AAC TAT GAT AGT AGA ACG TAT CCA ATT CGA ACA GTT
Leu Asp Ile Val Ser Leu Phe Pro Asn Tyr Asp Ser Arg Thr Tyr Pro Ile Arg Thr Val

                                                965                                               995
TCC CAA TTA ACA AGA GAA ATT TAT ACA AAC CCA GTA TTA GAA AAT TTT GAT GGT AGT TTT
Ser Gln Leu Thr Arg Glu Ile Tyr Thr Asn Pro Val Leu Glu Asn Phe Asp Gly Ser Phe

                                               1025                                              1055
CGA GGC TCG GCT CAG GGC ATA GAA GGA AGT ATT AGG AGT CCA CAT TTG ATG GAT ATA CTT
Arg Gly Ser Ala Gln Gly Ile Glu Gly Ser Ile Arg Ser Pro His Leu Met Asp Ile Leu
```

Tabelle 3: (Fortsetzung)

```
                                    1085                                                    1115
AAC AGT ATA ACC ATC TAT ACG GAT GCT CAT AGA GGA GAA TAT TAT TGG TCA GGG CAT CAA
Asn Ser Ile Thr Ile Tyr Thr Asp Ala His Arg Gly Glu Tyr Tyr Trp Ser Gly His Gln


                                    1145                                                    1175
ATA ATG GCT TCT CCT GTA GGG TTT TCG GGG CCA GAA TTC ACT TTT CCG CTA TAT GGA ACT
Ile Met Ala Ser Pro Val Gly Phe Ser Gly Pro Glu Phe Thr Phe Pro Leu Tyr Gly Thr


                                    1205                                                    1235
ATG GGA AAT GCA GCT CCA CAA CAA CGT ATT GTT GCT CAA CTA GGT CAG GGC GTG TAT AGA
Met Gly Asn Ala Ala Pro Gln Gln Arg Ile Val Ala Gln Leu Gly Gln Gly Val Tyr Arg


                                    1265                                                    1295
ACA TTA TCG TCC ACT TTA TAT AGA AGA CCT TTT AAT ATA GGG ATA AAT AAT CAA CAA CTA
Thr Leu Ser Ser Thr Leu Tyr Arg Arg Pro Phe Asn Ile Gly Ile Asn Asn Gln Gln Leu


                                    1325                                                    1355
TCT GTT CTT GAC GGG ACA GAA TTT GCT TAT GGA ACC TCC TCA AAT TTG CCA TCC GCT GTA
Ser Val Leu Asp Gly Thr Glu Phe Ala Tyr Gly Thr Ser Ser Asn Leu Pro Ser Ala Val


                                    1385                                                    1415
TAC AGA AAA AGC GGA ACG GTA GAT TCG CTG GAT GAA ATA CCG CCA CAG AAT AAC AAC GTG
Tyr Arg Lys Ser Gly Thr Val Asp Ser Leu Asp Glu Ile Pro Pro Gln Asn Asn Asn Val


                                    1445                                                    1475
CCA CCT AGG CAA GGA TTT AGT CAT CGA TTA AGC CAT GTT TCA ATG TTT CGT TCA GGC TTT
Pro Pro Arg Gln Gly Phe Ser His Arg Leu Ser His Val Ser Met Phe Arg Ser Gly Phe
```

EP 0 238 441 B1

Tabelle 3: (Fortsetzung)

```
                                                  1505                                                      1535
AGT AAT AGT AGT GTA AGT ATA ATA AGA GCT CCT ATG TTC TCT TGG ATA CAT CGT AGT GCT
Ser Asn Ser Ser Val Ser Ile Ile Arg Ala Pro Met Phe Ser Trp Ile His Arg Ser Ala


                                                  1565                                                      1595
GAA TTT AAT AAT ATA ATT CCT TCA TCA CAA ATT ACA CAA ATA CCT TTA ACA AAA TCT ACT
Glu Phe Asn Asn Ile Ile Pro Ser Ser Gln Ile Thr Gln Ile Pro Leu Thr Lys Ser Thr


                                                  1625                                                      1655
AAT CTT GGC TCT GGA ACT TCT GTC GTT AAA GGA CCA GGA TTT ACA GGA GGA GAT ATT CTT
Asn Leu Gly Ser Gly Thr Ser Val Val Lys Gly Pro Gly Phe Thr Gly Gly Asp Ile Leu


                                                  1685                                                      1715
CGA AGA ACT TCA CCT GGC CAG ATT TCA ACC TTA AGA GTA AAT ATT ACT GCA CCA TTA TCA
Arg Arg Thr Ser Pro Gly Gln Ile Ser Thr Leu Arg Val Asn Ile Thr Ala Pro Leu Ser


                                                  1745                                                      1775
CAA AGA TAT CGG GTA AGA ATT CGC TAC GCT TCT ACC ACA AAT TTA CAA TTC CAT ACA TCA
Gln Arg Tyr Arg Val Arg Ile Arg Tyr Ala Ser Thr Thr Asn Leu Gln Phe His Thr Ser


                                                  1805                                                      1835
ATT GAC GGA AGA CCT ATT AAT CAG GGG AAT TTT TCA GCA ACT ATG AGT AGT GGG AGT AAT
Ile Asp Gly Arg Pro Ile Asn Gln Gly Asn Phe Ser Ala Thr Met Ser Ser Gly Ser Asn


                                                  1865                                                      1895
TTA CAG TCC GGA AGC TTT AGG ACT GTA GGT TTT ACT ACT CCG TTT AAC TTT TCA AAT GGA
Leu Gln Ser Gly Ser Phe Arg Thr Val Gly Phe Thr Thr Pro Phe Asn Phe Ser Asn Gly


                                                  1925                                                      1955
TCA AGT GTA TTT ACG TTA AGT GCT CAT GTC TTC AAT TCA GGC AAT GAA GTT TAT ATA GAT
Ser Ser Val Phe Thr Leu Ser Ala His Val Phe Asn Ser Gly Asn Glu Val Tyr Ile Asp
```

EP 0 238 441 B1

EP 0 238 441 B1

```
                                        1985                                                    2015
CGA ATT GAA TTT GTT CCG GCA GAA GTA ACC TTT GAG GCA GAA TAT GAT TTA GAA AGA GCA
Arg Ile Glu Phe Val Pro Ala Glu Val Thr Phe Glu Ala Glu Tyr Asp Leu Glu Arg Ala


                                        2045                                                    2075
CAA AAG GCG GTG AAT GAG CTG TTT ACT TCT TCC AAT CAA ATC GGG TTA AAA ACA GAT GTG
Gln Lys Ala Val Asn Glu Leu Phe Thr Ser Ser Asn Gln Ile Gly Leu Lys Thr Asp Val


                                        2105                                                    2135
ACG GAT TAT CAT ATT GAT CAA GTA TCC AAT TTA GTT GAG TGT TTA TCT GAT GAA TTT TGT
Thr Asp Tyr His Ile Asp Gln Val Ser Asn Leu Val Glu Cys Leu Ser Asp Glu Phe Cys


                                        2165                                                    2195
CTG GAT GAA AAA AAA GAA TTG TCC GAG AAA GTC AAA CAT GCG AAG CGA CTT AGT GAT GAG
Leu Asp Glu Lys Lys Glu Leu Ser Glu Lys Val Lys His Ala Lys Arg Leu Ser Asp Glu


                                        2225                                                    2255
CGG AAT TTA CTT CAA GAT CCA AAC TTT AGA GGG ATC AAT AGA CAA CTA GAC CGT GGC TGG
Arg Asn Leu Leu Gln Asp Pro Asn Phe Arg Gly Ile Asn Arg Gln Leu Asp Arg Gly Trp


                                        2285                                                    2315
AGA GGA AGT ACG GAT ATT ACC ATC CAA GGA GGC GAT GAC GTA TTC AAA GAG AAT TAC GTT
Arg Gly Ser Thr Asp Ile Thr Ile Gln Gly Gly Asp Asp Val Phe Lys Glu Asn Tyr Val


                                        2345                                                    2375
ACG CTA TTG GGT ACC TTT GAT GAG TGC TAT CCA ACG TAT TTA TAT CAA AAA ATA GAT GAG
Thr Leu Leu Gly Thr Phe Asp Glu Cys Tyr Pro Thr Tyr Leu Tyr Gln Lys Ile Asp Glu
```

Tabelle 3: (Fortsetzung)

```
                              2405                                         2435
TCG AAA TTA AAA GCC TAT ACC CGT TAC CAA TTA AGA GGG TAT ATC GAA GAT AGT CAA GAC
Ser Lys Leu Lys Ala Tyr Thr Arg Tyr Gln Leu Arg Gly Tyr Ile Glu Asp Ser Gln Asp


                              2465                                         2495
TTA GAA ATC TAT TTA ATT CGC TAC AAT GCC AAA CAC GAA ACA GTA AAT GTG CCA GGT ACG
Leu Glu Ile Tyr Leu Ile Arg Tyr Asn Ala Lys His Glu Thr Val Asn Val Pro Gly Thr


                              2525                                         2555
GGT TCC TTA TGG CCG CTT TCA GCC CCA AGT CCA ATC GGA AAA TGT GCC CAT CAT TCC CAT
Gly Ser Leu Trp Pro Leu Ser Ala Pro Ser Pro Ile Gly Lys Cys Ala His His Ser His


                              2585                                         2615
CAT TTC TCC TTG GAC ATT GAT GTT GGA TGT ACA GAC TTA AAT GAG GAC TTA GGT GTA TGG
His Phe Ser Leu Asp Ile Asp Val Gly Cys Thr Asp Leu Asn Glu Asp Leu Gly Val Trp


                              2645                                         2675
GTG ATA TTC AAG ATT AAG ACG CAA GAT GGC CAT GCA AGA CTA GGA AAT CTA GAA TTT CTC
Val Ile Phe Lys Ile Lys Thr Gln Asp Gly His Ala Arg Leu Gly Asn Leu Glu Phe Leu


                              2705                                         2735
GAA GAG AAA CCA TTA GTA GGA GAA GCA CTA GCT CGT GTG AAA AGA GCG GAG AAA AAA TGG
Glu Glu Lys Pro Leu Val Gly Glu Ala Leu Ala Arg Val Lys Arg Ala Glu Lys Lys Trp


                              2765                                         2795
AGA GAC AAA CGT GAA AAA TTG GAA TGG GAA ACA AAT ATT GTT TAT AAA GAG GCA AAA GAA
Arg Asp Lys Arg Glu Lys Leu Glu Trp Glu Thr Asn Ile Val Tyr Lys Glu Ala Lys Glu


                              2825                                         2855
TCT GTA GAT GCT TTA TTT GTA AAC TCT CAA TAT GAT AGA TTA CAA GCG GAT ACC AAC ATC
Ser Val Asp Ala Leu Phe Val Asn Ser Gln Tyr Asp Arg Leu Gln Ala Asp Thr Asn Ile
```

EP 0 238 441 B1

Tabelle 3: (Fortsetzung)

```
                                             2885                                                                    2915
GCG ATG ATT CAT GCG GCA GAT AAA CGC GTT CAT AGC ATT CGA GAA GCT TAT CTG CCT GAG
Ala Met Ile His Ala Ala Asp Lys Arg Val His Ser Ile Arg Glu Ala Tyr Leu Pro Glu

                                             2945                                                                    2975
CTG TCT GTG ATT CCG GGT GTC AAT GCG GCT ATT TTT GAA GAA TTA GAA GGG CGT ATT TTC
Leu Ser Val Ile Pro Gly Val Asn Ala Ala Ile Phe Glu Glu Leu Glu Gly Arg Ile Phe

                                             3005                                                                    3035
ACT GCA TTC TCC CTA TAT GAT GCG AGA AAT GTC ATT AAA AAT GGT GAT TTT AAT AAT GGC
Thr Ala Phe Ser Leu Tyr Asp Ala Arg Asn Val Ile Lys Asn Gly Asp Phe Asn Asn Gly

                                             3065                                                                    3095
TTA TCC TGC TGG AAC GTG AAA GGG CAT GTA GAT GTA GAA GAA CAA AAC AAC CAC CGT TCG
Leu Ser Cys Trp Asn Val Lys Gly His Val Asp Val Glu Glu Gln Asn Asn His Arg Ser

                                             3125                                                                    3155
GTC CTT GTT GTT CCG GAA TGG GAA GCA GAA GTG TCA CAA GAA GTT CGT GTC TGT CCG GGT
Val Leu Val Val Pro Glu Trp Glu Ala Glu Val Ser Gln Glu Val Arg Val Cys Pro Gly

                                             3185                                                                    3215
CGT GGC TAT ATC CTT CGT GTC ACA GCG TAC AAG GAG GGA TAT GGA GAA GGT TGC GTA ACC
Arg Gly Tyr Ile Leu Arg Val Thr Ala Tyr Lys Glu Gly Tyr Gly Glu Gly Cys Val Thr

                                             3245                                                                    3275
ATT CAT GAG ATC GAG AAC AAT ACA GAC GAA CTG AAG TTT AGC AAC TGT GTA GAA GAG GAA
Ile His Glu Ile Glu Asn Asn Thr Asp Glu Leu Lys Phe Ser Asn Cys Val Glu Glu Glu
```

EP 0 238 441 B1

Tabelle 3: (Fortsetzung)

```
                                  3305                                                       3335
GTA TAT CCA AAC AAC ACG GTA ACG TGT AAT GAT TAT ACT GCG ACT CAA GAA GAA TAT GAG
Val Tyr Pro Asn Asn Thr Val Thr Cys Asn Asp Tyr Thr Ala Thr Gln Glu Glu Tyr Glu


                                  3365                                                       3395
GGT ACG TAC ACT TCT CGT AAT CGA GGA TAT GAC GGA GCC TAT GAA AGC AAT TCT TCT GTA
Gly Thr Tyr Thr Ser Arg Asn Arg Gly Tyr Asp Gly Ala Tyr Glu Ser Asn Ser Ser Val


                                  3425                                                       3455
CCA GCT GAT TAT GCA TCA GCC TAT GAA GAA AAA GCA TAT ACA GAT GGA CGA AGA GAC AAT
Pro Ala Asp Tyr Ala Ser Ala Tyr Glu Glu Lys Ala Tyr Thr Asp Gly Arg Arg Asp Asn


                                  3485                                                       3515
CCT TGT GAA TCT AAC AGA GGA TAT GGG GAT TAC ACA CCA CTA CCA GCT GGC TAT GTG ACA
Pro Cys Glu Ser Asn Arg Gly Tyr Gly Asp Tyr Thr Pro Leu Pro Ala Gly Tyr Val Thr


                                  3545                                                       3575
AAA GAA TTA GAG TAC TTC CCA GAA ACC GAT AAG GTA TGG ATT GAG ATC GGA GAA ACG GAA
Lys Glu Leu Glu Tyr Phe Pro Glu Thr Asp Lys Val Trp Ile Glu Ile Gly Glu Thr Glu


                                  3605
GGA ACA TTC ATC GTG GAC AGC GTG GAA TTA CTT CTT ATG GAG GAA TAA
Gly Thr Phe Ile Val Asp Ser Val Glu Leu Leu Leu Met Glu Glu End
```

EP 0 238 441 B1